# EUROPEAN PATENT APPLICATION

(11) **EP 4 686 414 A1**
(43) Date of publication of application: **04.02.2026**
(21) Application number: 24192261.6
(22) Date of filing: 01.08.2024
(51) Int. Cl.: A23J 1/14, A01H 1/06, A23J 3/14, A23L 11/40, A23L 29/206, A23L 33/185, C12N 15/01, C12N 15/82

(54) **METHOD OF IMPROVING GELATION PROPERTIES OF PEA PROTEINS**

(71) Applicant: KWS SAAT SE & Co. KGaA, 37574 Einbeck (DE)
(72) Inventor: Ilsemann, Karen, 37574 Einbeck (DE); Moore, Nigel, 37574 Einbeck (DE); Molitor, Alexandra, 37574 Einbeck (DE); Bemm, Felix, 37574 Einbeck (DE); Hölscher, Timo, 37574 Einbeck (DE); Zaborowski, Adam, 37574 Einbeck (DE); Dietrich, Katrin, 37574 Einbeck (DE); Müller, Madlen, 37574 Einbeck (DE)
(74) Representative: Eisenführ Speiser

(57) **Abstract**

The present invention is directed to plant-based protein ingredients suitable for use in consumables, including vegetarian or vegan meat or dairy analogues. The plant-based proteins have desirable organoleptic properties stemming from improved methodologies in plant genetics.

## Description

### Technical Field

The present invention is directed to methods for providing an altered and favorably improved gelation pattern of plant-based protein ingredients suitable for use alternative consumables, including vegetarian or vegan meat or dairy analogues. The protein ingredients have desirable organoleptic and viscoelastic properties stemming from improved methodologies in plant genetics.

### Background

In view of an increasing consumer awareness regarding animal welfare and diet-associated illnesses, including obesity, type II diabetes, prevention of dementia and cardiovascular diseases, the development of healthier food products is of increasing interest and there is an ever-increasing consumer demand for plant-based food and food additives. In view of challenges related to climate change and a steady increase in the world population further increase the awareness related to ecological sustainability in the context of developing new seeds, planting and using the same.

Producers are thus facing the challenges of providing plant-based alternatives at scale that meet consumers' expectations in taste, texture, and other functional and organoleptic properties.

To meet these demands, several sources of plant-based proteins have been considered by producers, including but not limited to: soybeans; peas, chickpeas and split peas; cranberries; beans such as navy, pinto, adzuki, fava, lima, black, red kidney, and mung beans; pumpkin seed and seed from other squash; and grains such as rice, sorghum, and millet. Consumers look for clean-labelled, locally-sourced and sustainable ingredients, which factors are also considered by producers. Further, certain consumers do not wish to consume animal products, including milk from cows, at all in view of its animal origin, due to lactose intolerance, or due to dairy allergies and the like. They may also see potential environmental sustainability issues. The global consumable industry has a continuing need to find plant-based protein sources that meet these requirements and preferences.

Peas (*Pisum sativum* or *Pisum sativum* L.) are annual plants with a one-year life cycle belonging to the family of *Leguminosae.* Peas belong to the oldest cultivated crops and domestication of wild pea plants likely already began with the start of agriculture about 11,000 years ago. Pea plants contain symbiotic bacteria within their root system with the special ability to fix nitrogen from the atmosphere, making them a highly attractive source of protein which meets the preferences of locally grown, sustainable agricultural practices.

One major challenge of working with pea protein ingredients and isolates or concentrates and the like thereof as part of compositions in consumable applications has to do with inconsistent functional properties of commercially available pea proteins, especially viscoelastic properties such as gelation. Indeed, pea protein gelation differs significantly from the process known from whey protein and pea protein aggregation under thermal and/or mechanical treatment often leads to undesired aggregation. Only subtly variations in protein content can result in poor textural performance, melting profiles, structural integrity, and the like, making the use of pea proteins as a versatile plant-based protein source difficult at date.

The core storage proteins of peas can be categorized as globulins and albumins. Globulins represent the predominant fraction of pea proteins (~ 50 to 80% of total protein) and are divided into legumin, vicilin, and convicilin proteins. Legumin is a hexamer composed of acidic and basic subunits connected by disulfide bonds (-S-S-). Vicilin usually occurs as a trimer with monomers of 50 kDa each (Liang and Tang, 2013, https://doi.org/10.1016/j.foodhyd.2013.04.005). The protein is known to fragment into 33 kDa (αβ), 30 kDa (βγ), 19 kDa (α), 13.5 kDa (β), and 16 or 12.5 kDa (γ) subunits in a (denaturing) SDS-PAGE (O'Kane et al., 2004, https://doi.org/10.1021/jf035215h). In contrast to legumin, vicilin is less hydrophobic. Structurally, vicilin does not contain any disulphide bonds. However, dependent on genotype, vicilin may contain one or two cysteine groups, which show a potential to form covalent bonds during aggregation (Chihi et al., 2016, https://doi.org/10.1021/acs.jafc.6b00087). The third major globulin protein is convicilin, which shows high homology to vicilin. It forms tetramers of 290 kDa with monomers of ~ 70 kDa. The amino acid sequence building up a monomer contains one cysteine group (Shrestha et al., 2023, https://doi.org/10.1016/j.foodchem.2023.135464).

Regarding the food-relevant properties, it was described that vicilin protein fractions can undergo heat-induced gelation, whereas convicilin has a negative effect on gelation (O'Kane et al. 2004, *supra*)*.* Legumin has a better emulsion capacity than vicilin (Cserhalmi et al., 1998, Emulsifying properties, surface hydrophobicity and thermal denaturation of pea protein fractions. Acta Alimentaria, 27, 357-363). Albumin has the best emulsifying properties but does not exhibit effective gelation (Shanthakumar et al., 2022, https://doi.org/10.3390/molecules27165354). At date, however, there is no detailed information or a suitable pea line available that has a high gelation pattern. Such a pea, and methods of producing suitable pea protein compositions thereof, is of great interest for the food and cosmetic market, as these compositions can be used to prepare, *inter alia*, a meat of fish like texture with a certain viscoelastic stability that is lacking in reference pea ingredients in view of the anti-gelation properties of convicilin.

As a drawback for food production, all globulin proteins all have a strong tendency for aggregation, as the processing after harvest, including thermal and mechanical treatments, results in denaturation and primary and subsequent aggregation. This hampers the control of the gelation pattern. Pea protein synthesis in the plant is regulated by complex genetic mechanisms involving multigene families encoding various protein types, and the mechanisms also reflect the developmental and metabolic processes that occur during the lifecycle of the pea plant. Gelation is the most crucial characteristic of globular protein. For food production using pea proteins, it is of utmost importance to be in a position to regulate the globulin content, as the globulin content significantly influences the behavior of the final product. This is complicated in case the raw materials contain a varying composition of globulins, including convicilin, naturally varies. Further, a pea protein compositions for use in a liquid or in a solid products is inherently associated with different needs.

In 2019, a pea reference genome was published, describing a total of 40 seed protein storage genes along with 210 upstream regulatory motifs for these genes (Kreplak, et al., Nat. Genet. 51, 2019, https://doi.org/10.1038/s41588-019-0480-1), indicating that the underlying genetic framework controlling pea protein content is large and complex. The functional properties of individual storage proteins such as globulins and albumin have also been studied and published. However, at date no correlation has thus far been made between the complex genetic framework of the pea plant and the functional properties of pea proteins produced in pea plant.

Therefore, there is currently a great need in providing homogeneously and stable pea protein ingredients, isolates, flours and compositions showing a uniform and predictable gelation pattern, wherein, for food safety and sustainability purposes, the identification and generation of natural *Pisum sativum* plants with fruits having an optimum globulin content, specifically with a given vicilin::convicilin ratio naturally modifying the gelation and yielding pea protein isolates and compositions with a high degree of gelation, without the need of chemical functionalization and/or cumbersome downstream purification represents a strong need to provide healthy and well standardized pea protein compositions suitable for a variety of different food applications.

Further, there is a great need in providing raw materials for alternative and hybrid food products to contribute to a reduced energy consumption during food production in comparison to animal-based food products and to guarantee an alternative source of food materials for a balanced and healthy nutrition. To this end, there is a great need in providing methodologies to provide plant-based lines within a short period of time that produce fruits, seeds and materials that can be planted and cultivated in an environmentally friendly way and which are suitable as valuable basis for alternative nutrition.

### Summary of the Invention

### Brief Description of the Drawings

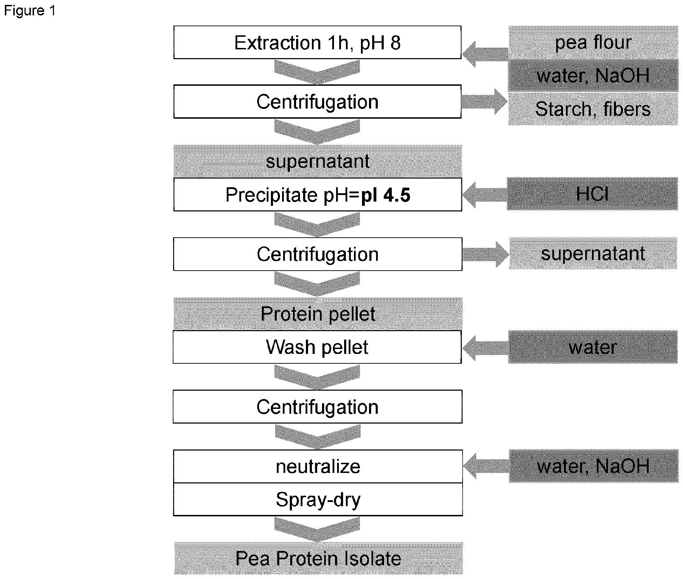

**Figure 1 (Fig. 1****)** shows a general and exemplary flow scheme for producing a pea protein isolate as further detailed in **Example 3.**

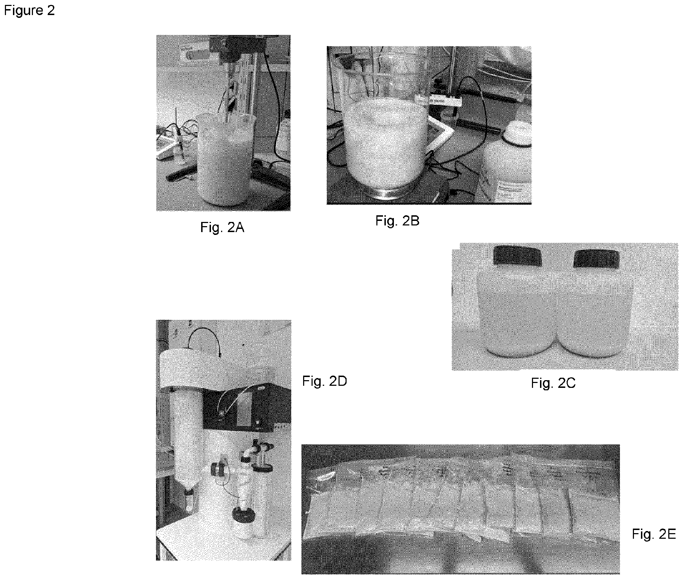

**Figure 2 (Fig. 2A to 2E****)** visualizes certain steps and products of the workflow detailed in **Fig. 1** **(****Fig. 2C** = protein pellet; **Fig. 2D** spray drying) until a protein isolate is obtained in **Fig. 2E** for various starting materials tested. Details are explained in **Example 3.**

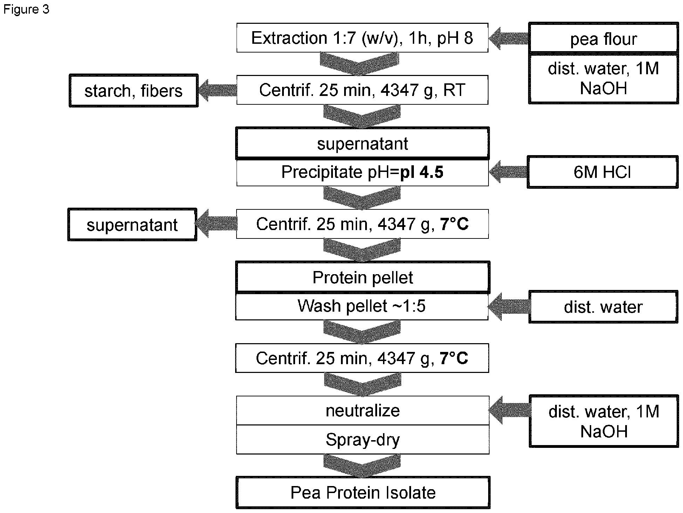

**Figure 3 (Fig. 3****)** provides a very specific workflow for the flow scheme as generally depicted in **Fig. 1****.** Details are again explained in **Example 3.**

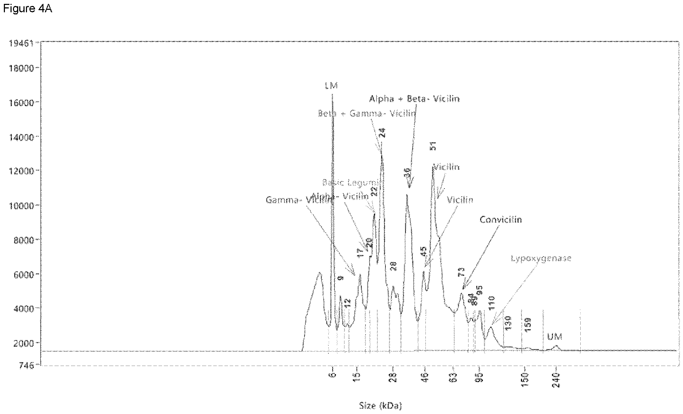

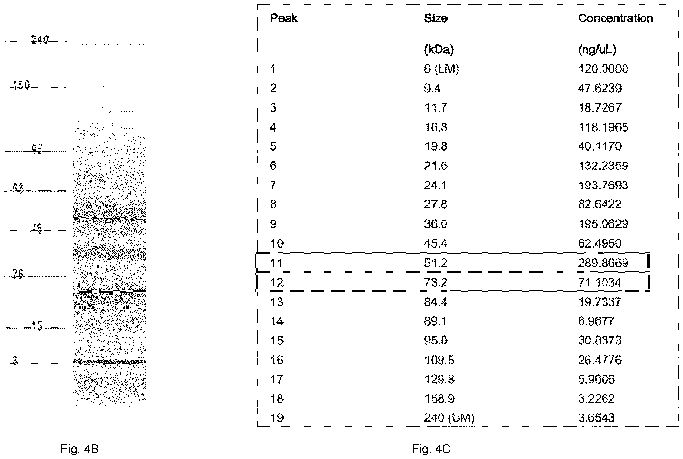

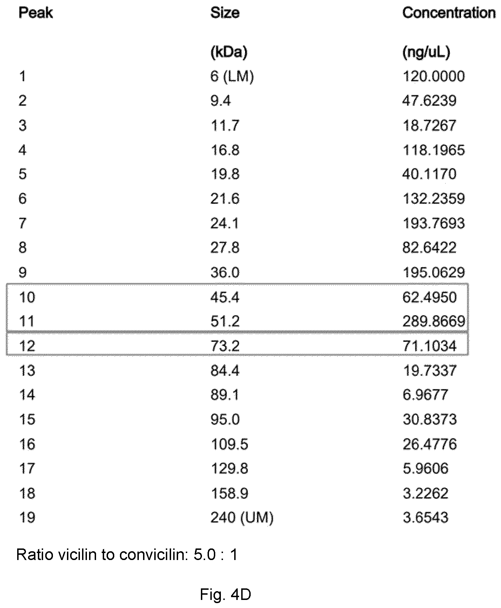

**Figure 4 (Fig. 4A to 4D****)** shows the results of a storage protein measurement for the line KWS 1 as detailed in **Example 4.** Notably, **Fig. 4A** shows the peaks obtained for the proteins of different size (kDa) measured and **Fig. 4B** shows the corresponding SDS-PAGE. **Fig. 4C** and **Fig. 4D** show the calculation of proteins obtained. **Fig. 4C** highlights vicilin with signal peptide (no. 11, 51.2 kDa) and convicilin with signal peptide (no. 12, 73.2 kDa), whereas **Fig. 4D** additionally marks vicilin without signal peptide (no. 10, 45.4 kDa). For vicilin to convicilin ratio determinations, all available forms as detectable and as highlighted in **Fig. 4D** were considered.

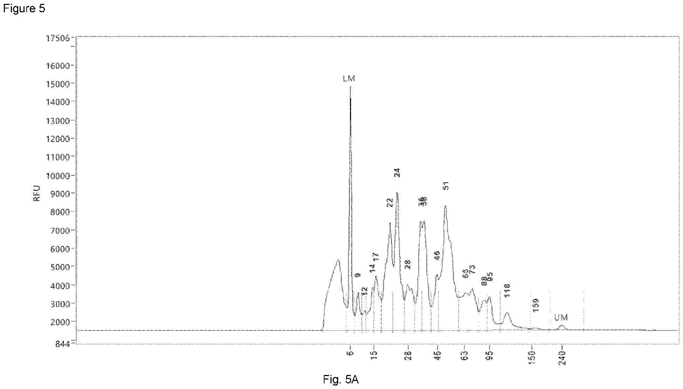

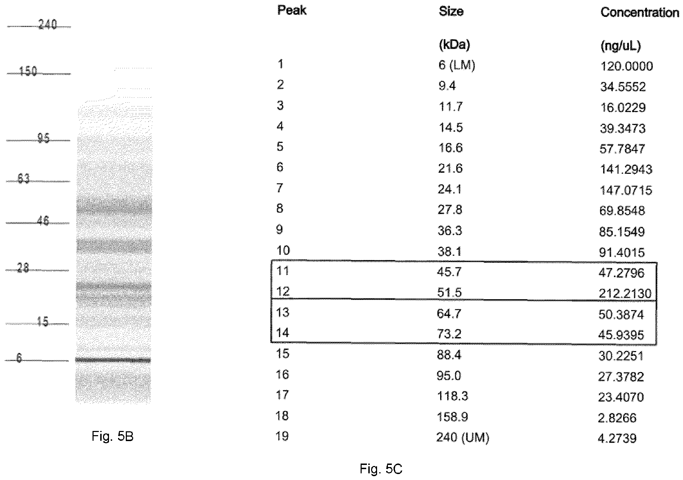

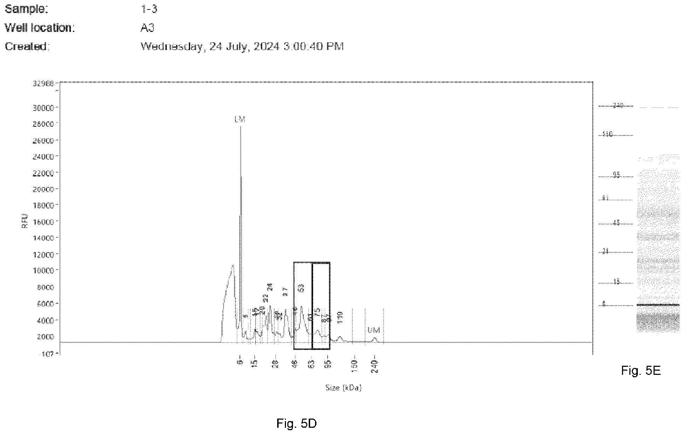

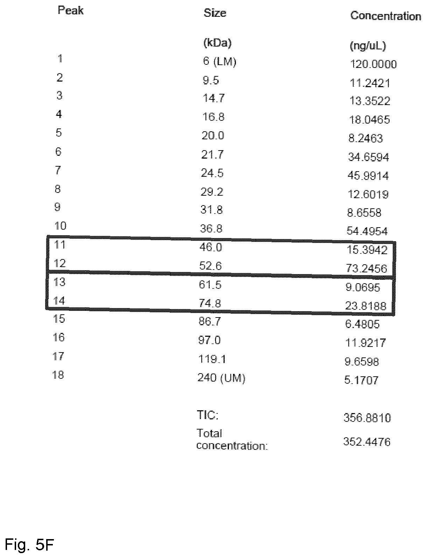

**Figure 5** **(****Fig. 5A to 5C****)** shows the results of a storage protein measurement for the line KWS 2 as detailed in **Example 4.** In all experiments for **Fig. 5A to 5F****,** vicilin and convicilin was identified both in the form with and without signal peptide. The sum of vicilins (without and with signal peptide) and the sum of convicilins (without and with signal peptide), whenever both species could be identified in a pea protein measurement was used to define vicilin to convicilin ratio as used herein. **Figure 5** **(****Fig. 5D to 5F****)** then shows the results of a storage protein measurement for the line Proklam as also detailed in **Example 4.** Also for this measurement, vicilin and convicilin was identified both in the form with and without signal peptide. The sum of vicilins (without and with signal peptide) and the sum of convicilins (without and with signal peptide), whenever both species could be identified in a pea protein measurement was used to define vicilin to convicilin ratio as used herein (see **Fig. 5F****).**

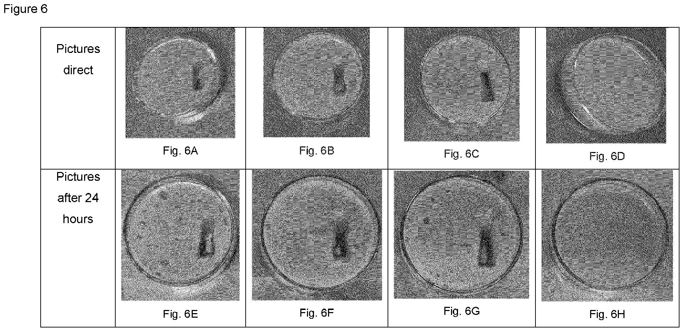

**Figure 6 (Fig. 6A** to **6H****)** provides the visualization of the agar ranking test as standardized gelling test as detailed in **Example 5** and corresponding to the measurements as shown in **Table 3** and providing a convenient tool to determine and compare the gelation properties and pattern of different gels obtained from different pea lines. Top row **(****Fig. 6A** to **6D****)** shows the direct measurement for KWS1, KWS2, KWS3, and KWS Proklam, respectively. The bottom row **(****Fig. 6E** to **6H****)** shows the follow-up measurement for KWS1, KWS2, KWS3, and KWS Proklam, respectively, after 24 h.

### Definitions

An "alternative consumable product ingredient" as used herein refers to an ingredient from a plant, including a protein ingredient, which is suitable as part of a "consumable product" or of a "consumable composition". Further, at least a part or fraction of the "alternative consumable product ingredient" is suitable as a substitute for a commonly known and commonly fabricated product ingredient, usually it is suitable as an alternative to, for example, animal-derived or animal-produced ingredients or food, including meat, eggs or dairy.

An "alternative food" or "alternative nutrition" or "alternative (food) product / composition" as used herein refers to a food, including liquid food like beverages, which is usually a plant- or microorganism-based food that is an alternative to animal-derived food, including meat or dairy. Alternative food products may be of particular interest as an alternative source of proteins, but the term alternative food refers to any kind of nutritional building block, including proteins, carbohydrates, lipids, vitamins, minerals, fibers and the like that are suitable for food production and that are well accepted or even healthy as food and feed for human beings or farm animals and pets. An alternative food product or alternative food thus represents an "alternative consumable product" or an ingredient thereof. The ingredients of the present invention are useful to be implemented into an alternative food, an alternative feed or an alternative cosmetic.

An "alternative food product" as used herein may be present in liquid form, in semi-liquid form or in solid form. As used herein, an "alternative food product" refers to a product for human or animal consumption that is usually made with ingredients from animal sources, but in which the animal-sourced ingredient has been partially or fully replaced with a plant-based substitute ingredient. Non-limiting examples of alternative food products include alternative beverages such as a milk substitute or a drinkable yogurt substitute, or alternative food products such as an egg, beef, dairy, poultry, or seafood substitute. Other alternative food products include pet or animal feed products in which some or all of the animal-sourced ingredients are substituted with plant-based ingredients. The replacement or substitution of the animal-sourced ingredient may in some embodiments be e.g. more than 70%, more than 80%, more than 90%, or more than 95%. In other embodiments, the replacement or substitution of the animal-sourced ingredient may be 70% or less, such as 60%, 50%, 40%, etc. Alternative food products may also include non-dairy beverages such as sports drinks or smoothies. Alternative food products may further refer to alternative nutritional products, such as plant-based powder, to be used as dietary or nutritional supplements.

An alternative product or an alternative composition as used herein, and any pea protein ingredient suitable for the production thereof of the present invention is specifically processed (industrially and/or mechanically and/or chemically and/or enzymatically) and a pea protein ingredient of this invention will usually be processed, isolated, concentrated and/or otherwise treated for inclusion in an alternative product or composition. Additionally a pea protein ingredient of this invention will usually represent an intermediate ingredient, that was or that can be isolated from a plant representing one part or fraction of a final product or composition, or of a mixture or hybrid product. In an alternative product or composition, the pea protein ingredient of this invention as alternative part or fraction of the alternative product or composition thus substitutes a part or fraction that would be present in a commonly known and commonly fabricated product or composition, preferably, wherein it substitutes a part or fraction of animal or non-plant origin in the corresponding commonly known and commonly fabricated product.

Whenever the terms "composition" or "pea protein composition" is used herein, it refers to a composition of any of the protein ingredients, flours, concentrates or isolates disclosed herein that are used in an extracted form together with other ingredients from different origins to provide said composition. An alternative food or a cosmetic is thus also a composition in this sense. In this context, a "mixture" or "pea protein mixture" is a specific form of a composition, wherein pea protein fractions from different peas or even from different plants or other sources are mixed with each other to provide a basic protein mixture (and optionally further additives or ingredients of different nature and/or origin) comprising pea protein of the present invention.

A "consumable product" as used herein refers to goods that are usually understood to be used up or depleted during normal business operations, such as food and beverage, office supplies, cleaning and sanitary products, and medical supplies. In line with the general understanding, there are two main types of consumables: durable consumables, which are expected to last over a long period of time, and non-durable consumables, which are expected to be used up relatively quickly. Examples of consumables include perishable foods and beverages, paper products, ink cartridges, cleaning chemicals, gloves, and syringes. The consumable products particularly dealt with herein are non-durable consumables that are non-toxic when swallowed or applied on the human or animal body based on their intended use that are usually made of at least one organic raw materials (and optionally others), including food, beverages, gels, ointments, tooth paste and the like.

A "consumable product /composition" according to the present invention represents an alternative product or composition comprising at least one alternative plant-based ingredient, preferably a protein ingredient, according to the present invention, preferably a pea-based ingredient.

As used herein, "dairy substitute" or "dairy substitute composition" or "dairy alternative" or "dairy alternative product" refer to compositions that mimic the general appearance, nutritional content, and/or taste of dairy products produced using animal milk products without containing animal-based milk or being substantially free of animal-based products, and includes hybrid products made with lab-grown, fermented and animal-based components such as protein components. The dairy substitute may be completely free of any animal-based milk or animal-based milk protein or almost free of any animal-based milk protein, such as e.g. 90% free, or 95% free of any animal-based milk protein. The dairy substitute may be a dairy-free cheese, a dairy-free yogurt, a dairy-free ice cream, and the like.

Notably, the term "food" as used herein refers to a food intended for human nutrition, whereas a "feed" as used herein refers to a feed.

As used herein, "meat substitute" or "meat substitute composition" or "meat alternative" refers to compositions that mimic the general, organoleptic, and/or nutritional properties of consumable products produced using any type of meat or meat analog, including meat, fish, poultry, lab-grown and fermented meat products. This definition includes hybrid products made with lab-grown, fermented and animal-based components, such as protein components. A similar definition is used herein for "egg substitute", "egg substitute composition," and "egg alternative".

A "gene" as used herein refers to the coding region of a gene, the non-coding region as well as upstream and/or downstream located regulatory sequences, including an enhancer, silencer, promoter elements (e.g., proximal, distal and core promoter elements), and 5' and/or 3'UTRs. Therefore, a modification of a gene may also include the modification of a non-coding and/ or of a regulatory sequence thereof.

A "hybrid composition / product" or a "hybrid alternative (consumable, including food/cosmetic etc.) composition / product" as used herein refers to an alternative product that at least partially comprises an "alternative food" or "alternative nutrition" or "alternative (food) product" comprising a plant-originating protein substance according to the present invention, but which may comprise further ingredients.

A "knock-down" of a gene refers to an experimental technique by which the expression of the gene (i.e., the transcription from DNA to RNA and thus the amount of active RNA transcripts) is reduced. A reduced expression can e.g., be achieved by gene silencing reducing or abolishing the transcription rate and thus decreasing the amount of functional RNA available.

A "knock-out", on the other hand, leads to an abolished expression (transcription / translation), i.e., the gene is not fully or wrong transcribed so that expression is abolished at all or (on protein level) to such an extent that nearly no functional protein is expressed, or is expressed very level at or about the detection limit. This can e.g., be achieved by replacing or interrupting the sequence of the target gene. For example, an early or premature additional stop codon, preferably close to the start codon can lead to a premature transcription stop that results in the complete loss of functionally translated protein. Alternatively, a "knock-out" can be achieved by a mutation leading to a variation in the naturally occurring splice donor or splice acceptor site of a eukaryotic gene comprising exons and introns. The splice donor site usually includes an almost invariant sequence GU at the 5' end of the intron, within a larger, less highly conserved region. The splice acceptor site at the 3' end of the intron terminates the intron with an almost invariant AG sequence.

If these conserved sequences are mutated, RNA splicing is modified, which may also result in a knock-out as measured on a transcript (RNA) or translation (protein) level. Consequently, a single targeted nucleotide exchange, deletion or insertion may result in a functional knock-out in the sense that the sequence encoded by a gene is no longer transcribed or translated. A "knock-out" may also be produced by a deletion of a gene, or of a substantial part thereof, on a genomic level, optionally accompanied by a substitution against another sequence.

A "mutation" or a "genome modification" in the context of the present invention refers to any change of a coherent nucleic acid sequence by modifying a nucleic acid sequence at a given nucleotide sequence position that results in at least one difference in the (nucleic acid) sequence distinguishing it from the original sequence. In particular, a modification can be achieved by insertion or addition of one or more nucleotide(s), or substitution or deletion of one or more nucleotide(s) of the original sequence or any combination of these.

A "nucleic acid construct", "construct" or "expression construct" refers to a nucleic acid molecule encoding or comprising one or more genetic elements, which upon introduction into a target cell can be transcribed and/or translated into a functional form, e.g., RNA(s) or polypeptide(s) or protein(s). A nucleic acid construct may also comprise regulatory sequences such as promoter and terminator sequences facilitating expression of the genetic element(s) as well as spacers and introns. The genetic elements of the present invention can also be encoded on a set of constructs, which constructs can be introduced into a cell simultaneously or consecutively.

A "(pea) protein flour" as used herein refers to an ingredient that contains milled peas.

"Protein texturate" or "textured protein" as used herein refers to an ingredient having a structural integrity and identifiable structure such that individual units, appearing as fibers, shreds, chunks, bits, granules, slices, and the like, will withstand hydration and cooking or other procedures used in the production of food for consumption. In general, textured proteins may be used to alter or enhance texture and bind water. Edible protein sources from which textured proteins are produced may include, but are not limited to, legumes (e.g., pulse protein), pea, soy, com, wheat, chickpea, potato, rice, sunflower, and the like. Textured proteins may include, but are not limited to, textured pea protein, textured soy flour, textured soy concentrate, textured wheat protein, textured potato protein, or combinations thereof. Methods for protein texturization are known and described in the art, and may include, for example, high temperature and pressure extrusion, spinning, freeze texturization, chemical or enzymatic texturization, and the like.

The term "RNAi" or "RNA silencing" or "gene silencing" as used herein interchangeably refer to the process called RNA interference meaning a gene down-regulation (or knock-down) mechanism meanwhile demonstrated to exist in all eukaryotes. The mechanism was originally recognized and described in plants where it was called "post-transcriptional gene silencing" or "PTGS". In RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". In an RNA hairpin, sense sequences and corresponding antisense sequences, together form a double strand and are separated by an "intervening intron loop sequence" forming the loop of the hairpin structure.

The terms *Pisum sativum* (L.) plant and pea plant, and short only pea, are used interchangeably herein, wherein the term pea is used in the context of the plant as a whole, but also to denote parts thereof, particularly seeds/fruits within pea pods.

A "pea plant ingredient" as used herein is to be understood as the total amount of protein that can be extracted from a pea fruit or seed (dry or fresh). A "(pea) protein ingredient" is in turn to be understood as the total amount of protein of the (pea) plant.

The term "protein concentrate" is a protein ingredient with a concentration of about 30% to 60%. A "protein isolate" is an even more concentrated protein ingredient with a concentration of about 60% to about 100%. A "protein flour" represent the protein that can be obtained directly after dehulling and milling. As this protein flour is not yet heavily processed, it reflects the original content of protein ingredients rather directly. Therefore, the "protein flour" was also used to define standard ratios by the inventors when comparing different material herein below. A "protein texturate" or "texturized vegetable protein" is used to describe a usually further defatted flour product that is particularly suitable and used as a meat analogue or meat extender. It is quick to cook, with a protein content comparable to some meats. The terms "protein flake" or "protein powder" further describe the form of the protein. A "protein powder" is usually composed of fine, dry particles produced by the grinding, crushing, or disintegration of a solid substance.

The term "vector" refers to an element used for introducing a nucleic acid construct or set of nucleic acid constructs into a cellular system. The vector may be a plasmid or plasmid vector, cosmid, artificial yeast artificial chromosomes (YAC), bacterial artificial chromosome (BAC) or P1 artificial chromosomes (PACs), phagemid, bacterial phage based vector, a modified viral vector, an Agrobacterium shuttle vector, an isolated single-stranded or double-stranded nucleic acid sequence, comprising DNA and RNA sequences in linear or circular form, or a mixture thereof, for introduction or transformation into a plant, plant cell, tissue, organ, or material according to the present disclosure.

The terms "plan" or "plant cell" or "part of a plant" as used herein refer to a plant organism, a plant organ, differentiated and undifferentiated plant tissues, plant cells, seeds, and derivatives and progeny thereof. Plant cells include without limitation, for example, cells from seeds, from mature and immature cells or organs, including embryos, meristematic tissues, seedlings, callus tissues in different differentiation states, leaves, flowers, roots, shoots, male or female gametophytes, sporophytes, pollen, pollen tubes and microspores and protoplasts etc.

"Mutagenesis" refers to a technique, by which modifications or mutations are introduced into a nucleic acid sequence in a random or non- site-specific way. For example, mutations can be induced by certain chemicals such as EMS (ethyl methanesulfonate) or ENU (N-ethyl-N-nitrosourea) or physically, e.g., by irradiation with UV or gamma rays. "Site-specific modifications", on the other hand, rely on the action of site-specific effectors such as nucleases, nickases, recombinases, transposases, base editors, prime editors and the like. These tools recognize a certain target sequence and allow to introduce a modification at a specific location within the target sequence.

A "protein composition" as used herein refers to a protein isolate directly obtainable from a fruit, seed, particularly from a specific pea of the genus *Pisum,* or to a flour, a protein fraction, a purified or partially purified protein fraction. The protein composition, depending on the way of preparing the same, may include denatured and/or partially fragmented proteins, as the proteins may have undergone denaturation and/or fragmentation during thermal, chemical and/or mechanical processing/purification. A protein composition may consist of substantially one protein or a fragment thereof, particularly convicilin, or it may be a protein composition mixture comprising other proteins, particularly globulins and further pea proteins.

"TILLING" (Targeting Induced Local Lesions in Genomes) is a process, which allows to identify mutations in a specific gene after an (unspecific) mutagenesis has been performed. Mutagenesis may e.g., be performed using a chemical mutagen such as EMS. Then, a sensitive DNA screening technique is used to identify single base mutations. Methods for performing TILLING are known to the skilled person.

A "functional homolog(ue)" as used herein refers to a molecule having substantially the same function as a reference molecule. A "structural homolog(ue)" refers to a homolog having a substantial degree of sequence identity to a reference molecule, or a part thereof it originates from.

Whenever the present disclosure relates to the percentage of identity of nucleic acid or amino acid sequences to each other, these values define those values as obtained by using the EMBOSS Water Pairwise Sequence Alignments (nucleotide) programme (www.ebi.ac.uk/Tools/psa/emboss_water/) nucleic acids or the EM-BOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequence" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimize" if the respective sequence is to be used in another organism in comparison to the original organism a molecule originates from.

### Description of the Invention

The present invention is directed to improvement of the viscoelastic properties of pea proteins, in particular gelation, and identifying and optimizing the pea plant genetics that are involved in obtaining desirable viscoelastic performance of pea proteins, namely in the provision of pea protein ingredients having an increased gelation pattern in comparison to standard pea lines making the pea protein ingredients highly attractive for alternative food and cosmetics production, especially for products and compositions, where a strong texture and an adjustable good gelation is needed. Particularly, the present invention is based on the finding that specific variants of the protein convicilin and a certain ratio of vicilin to convicilin can favorably influence the gelation pattern of a pea protein mixture of a pea plant so that alternative foods and consumable products can be made with specific pea lines characterized by a favorable convicilin content. Further, methods of screening for an providing suitable pea lines are provided strongly facilitating the provision of pea protein ingredients with a standardized and predictable altered gelation pattern.

In one aspect, there is thus provided a method of altering a gelation pattern of the pea protein ingredient of a *Pisum sativum* plant, the method comprising the following steps: (i) providing at least one pea comprising at least one nucleic acid molecule comprising at least one modification of a nucleotide sequence of at least one gene, including the regulatory region thereof, wherein the at least one gene modified encodes a convicilin protein, wherein said at least one modification affects the gelation pattern of the pea protein ingredient, wherein said at least one modification affects at least one nucleotide sequence of a Psat06G0558100 reference gene (SEQ ID NO: 2) in comparison to the variant Psat06G0558100 reference gene (SEQ ID NO: 1) and/or of a Psat06G0558200 reference gene (SEQ ID NO: 66) in comparison to the variant Psat06G0558200 reference gene (SEQ ID NO: 65), or of a combination thereof; (ii) optionally: cultivating the *Pisum sativum* plant to obtain at least one pea comprising a protein composition having an altered gelation pattern in comparison to a *Pisum sativum* plant not comprising the at least one modified convicilin gene, or the regulatory region thereof; and (iii) obtaining a pea protein ingredient of said *Pisum sativum,* wherein said pea protein ingredient obtained, preferably as intermediate, has an altered gelation pattern, wherein the altered gelation pattern is defined as an altered vicilin to convicilin ratio in a pea protein ingredient, preferably in a pea flour, in comparison to a reference non-processed vicilin to convicilin ratio of 2:1 to 4:1 (wt./wt.) of a pea protein ingredient, preferably a flour, of a reference *Pisum sativum* plant, wherein the altered vicilin to convicilin ratio is characterized by a decreased amount of convicilin in comparison to the amount measured for the reference *Pisum sativum* plant and thus an increased vicilin to convicilin ratio. Preferably, the above method is not an essentially biological method, as at least one non-naturally occurring SNP, or gene or portion thereof has to be introduced into a reference gene.

Notably, the present inventors surprisingly found that by modifying the amount of total convicilin in a pea and particularly the ratio of vicilin and convicilin resulting from this targeted modification has a significant impact on the gelation, i.e., the viscoelastic properties, of the total protein ingredients obtainable from a pea by defining, providing and testing the relevant modified variants that show a reduced or even abolished transcription and/or translation of the (usually two, or more) genomic copies of convicilin. Altering in the context of the present invention thus preferably means a reduction of convicilin and thus an increase of gelation properties of the protein ingredients, optionally processed and purified, when used in various compositions, preferably when used in alternative food, where high-value proteins from plants with excellent texture and stability are of great interest.

Vicilin, as all pea globulins, comprises a signal peptide for import into the endoplasmic reticulum (ER), which is later on co-translationally removed. Vicilins have two cleavage sites, depending on the isoform (A and B. Cleavage yields fragments of about 20 kDa (alpha), 13 kDa (beta) and 12-16 kDa (gamma). Cleavage at site A yields fragments of about 20 kDa (alpha) and 25-30 kDa (beta + gamma), whereas cleavage at site B yields fragments of about 30-36 kDa (alpha+ beta) and 12-16 kDa (gamma). For convicilins, no post- or co-translational modifications other than the removal of the signal peptide also comprised for this pea globulin was described (source: Tzitzikas *et al.*, 2006, doi: 10.1021/jf0519008).

During testing of a plethora of pea flours of various origin, it turned out that - depending on the stage of harvesting - vicilin and convicilin were usually present in the pea material tested with and without signal peptide. As the gelation properties of the proteins with and without signal peptide are not significantly influenced by the signal peptide, the present inventors established a robust and easily reproducible test to measure the gelation of pea-derived proteins, usually in a less processed pea flour composition, and identified that the ratio of total vicilin (i.e., vicilin with and without signal peptide) to total convicilin (with and without signal peptide) represents a reliable indicator to measure the gelation strength and thus the gelation pattern of a given pea material having a certain convicilin-expression signature. To have a uniform basis of calculation (given the fact that particularly convicilin was identified in the signal peptide fused form only in certain lines, see **Fig. 5****),** it was decided that the vicilin to convicilin ratio as used herein thus represents the sum of total vicilin and/or convicilin respectively, without cleavage products (for vicilin), but always caluculating the sum of vicilin and convicilin both with and without the signal peptide (cf. **Fig. 4C****,** **Fig. 4D** and **Fig. 5C****:** peaks at ~ 45 kDa = vicilin without signal peptide and ~ 51 kDa correspond to vicilin with signal peptide the sum of which is used to calculate total vicilin; whereas for convicilin used in determining the ration corresponds to peaks a about 64.7 kDa and 73.2 kDa without and with signal peptide, respectively, **Fig. 5C** showing both convicilin forms as no. 13 and 14, respectively, for KWS line 3, whereas for KWS line 1, only the convicilin with signal peptide was identified as no. 12 (73.2 kDa) in **Fig. 4C** and **Fig. 4D****).** As the calculation of total vicilin / convicilin with and without signal peptide (but not including potential cleavage forms not frequently identified during early processing when producing pea flour test samples) is easier to reproduce and showed to be more robust, the vicilin to convicilin ratio as disclosed herein is the ratio of vicilin to convicilin measured in total protein or protein flour fractions of pea and determined as detailed in the Examples and as represented for exemplary measurements in the Figures **(****Fig. 4** and **Fig. 5****).**

The altered gelation pattern as achieved by modifying the convicilin amount in a pea protein ingredient based on the teachings of the present invention thus represents a favorable plant trait of interest, as the respective plants are highly suitable for providing proteins useful for making alternative food or stabilizers in cosmetics having a natural origin.

In one embodiment of the above aspect, there is provided a method, wherein the amount of convicilin in the pea protein ingredient, preferably in the pea flour, is reduced by at least 5%, at least 10%, at least 15%, at least 20%, at least 25%, preferably at least 30%, at least 35%, at least 40%, at least 45%, at least 50%, at least 55%, at least 60%, at least 70%, more preferably at least 75%, at least 80%, at least 85%, at least 90%, at least 95%, at least 100%, even more preferably at least 200%, at least 250%, at least 300%, at least 400%, at least 500%, and most preferably almost completely lost in comparison to the amount measured for the reference *Pisum sativum* plant.

According to the various aspects and embodiments of the present invention, there are provided sequences and methods to obtain a *Pisum sativum* plant with a convicilin pea protein encoding sequence that can be modified in a targeted way depending on the gelation pattern needed. The present invention provides a scheme to modify convicilin genes / alleles in a step-by-step way to achieve an adjustable decrease of convicilin expression that is correlated with an increased gelation to provide suitable pea protein ingredients with a desired gelation pattern.

The reference *Pisum sativum* plant as also characterized herein below in direct comparison to pea lines established and tested according to the present invention is the pea line Proklam that is publicly available and well characterized.

All Psat locus information provided herein is based on the nomenclature and annotation as presented by Yang et al., Nat. Genet., 2022, https://doi.org/10.1038/s41588-022-01172-2). Psat06G0558100 (SEQ ID NO: 1), for example, lies on chromosome 6 of the reference genome line ZW6 between position 448208288 and 448211033 based on the annotation of said reference genome.

The method of claim 1 or 2, further comprising a step of processing, preferably including a step of extraction, and/or purifying the pea protein ingredient of said *Pisum sativum* plant having an altered gelation pattern, wherein the processing preferably at least includes a step of dehulling, to obtain a protein flour, a protein concentrate, a protein isolate, a pea protein texturate, a pea protein powder, or a pea protein flake of a *Pisum sativum* plant.

In one embodiment, there is provided a method according to the above first aspect, wherein the convicilin protein has a sequence selected from any one of SEQ ID NOs: 121 and 122, or a sequence having at least 85%, 86%, 87%, 88%, 89%, 90%, 91%, 92%, 93%, 94%, 95%, 96%, 97%, 98%, or at least 99% sequence identity thereto, and/or wherein the vicilin protein preferably has a sequence of SEQ ID NO: 123 or a sequence having at least 85%, 86%, 87%, 88%, 89%, 90%, 91%, 92%, 93%, 94%, 95%, 96%, 97%, 98%, or at least 99% sequence identity thereto. As detailed above, and as it is known to the skilled person, other vicilin and convicilin analogoues, homologues, orthologues and paralogues and, particularly for vicilin, smaller cleavage products may exist. SEQ ID NO: 123 thus exclusively serves as reference sequence for one representative vicilin protein from *Pisum sativum* not cleaved into its subunits.

In yet another embodiment, there is provided a method, wherein the at least one modification is a deletion, substitution, or an insertion, preferably a targeted substitution, of at least one nucleotide sequence of the at least one gene, including the regulatory region thereof, encoding the convicilin protein.

In one embodiment of the methods as described herein, the at least one modification is provided by a step of mutagenesis, wherein mutagenesis includes chemical mutagenesis, radiation mutagenesis, and genome editing, wherein genome editing includes editing by site-directed nucleases, including zinc-finger nuclease (ZFNs) systems, transcription activator-like effector nuclease (TALENs) systems, meganuclease systems and CRISPR/Cas systems, preferably wherein mutagenesis is resulting in a knock-down, more preferably a knock-out of at least one gene encoding convicilin.

According to the embodiments as disclosed herein, site-directed and random mutagenesis are thus encompassed by the term mutagenesis. Mutagenesis may also refer to natural mutagenesis occurring as natural phenomenon. Based on the screening and selection methods as provided herein, such mutagenesis events in a convicilin gene can be easily determined to identify interesting germplasm carrying at least one signature mutation according to the present invention.

According to all aspects of the present disclosure, the *Pisum sativum* gene encoding convicilin may be located on any chromosome of a *Pisum sativum* plant. The relevant gene region can be easily identified. The altered expression of the gene or genes can be achieved by mutagenesis, TILLING, or genetic engineering. Identification and selection of plants comprising genes with an altered expression can be performed using marker-assisted selection using random or functional DNA markers based on the sequence and the position information provided herein.

In one alternative aspect, there is provided a method of producing a plant of the genus *Pisum,* preferably *Pisum sativum,* having an improved gelation pattern in view of the decreased convicilin amount expressed and an increased vicilin to convicilin ratio according to the first aspect, the method comprising the (a) introduction of; and/or (b) mutagenesis to confer, preferably chemical- or radiation-induced mutagenesis, and/or targeted mutagenesis, at least one functional copy of the modified nucleotide sequence selected from any one of SEQ ID NOs: 3 to 64 or 1, having at least one modification in comparison to the reference gene sequence of SEQ ID NO: 2, and/or at least one functional copy of the modified nucleotide sequence is selected from any one of SEQ ID NOs: 67 to 120, or 65 having at least one modification in comparison to the reference gene sequence of SEQ ID NO: 66, or any combination the aforementioned modifications of SEQ ID NOs: 3 to 64 and/or SEQ ID NOs: 67 to 120, or a nucleotide sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99% sequence identity to the respective sequence provided that the respective signature mutation as defined in **Table 1** and **Table 2** is still present, or a nucleic acid molecule comprising at least one modification of a nucleotide sequence of at least one gene, including the regulatory region thereof, the gene encoding a convicilin protein, wherein the modified nucleotide sequence comprises at least one signature mutation as defined in Table 1 and Table 2 in comparison to the reference gene sequence of SEQ ID NO: 2 or SEQ ID NO: 66, respectively, into a *Pisum* plant, preferably a *Pisum sativum* plant of interest.

As detailed in the Examples, a deep and thorough screen for single nucleotide polymorphisms (SNPs) in the convicilin gene (all alleles; various reference genomes screened) was performed to identify suitable modifications. These were screened for, optionally inserted in a targeted way into test lines, or lines carrying suitable germplasm as identified after screening were tested. A clear correlation of the SNP mutations and the desired high gelation pattern due to a decrease in the amount of convicilin proteon transcribed or expressed was surprisingly confirmed. Suitable SNPs, reference positions and the corresponding SEQ ID NOs are shown in **Tables 1** and **2** for Psat06G0558100 **(Table 1)** and Psat06G0558200 **(Table 2)** respectively. The SNP position is indicated as exact position in the reference genome.

**Table 1**

| SEQ ID NO: | Position KE_ZW6_hq-v1.chr6 Psat06G0558100 | Reference | Modification | Effect (genomic level) | Effect (protein level) |
|---|---|---|---|---|---|
| 3 | 448208301 | A | G | start_lost | +++ |
| 4 | 448208303 | A | G | stop_gained | +++ |
| 5 | 448208304 | A | G | stop_gained | +++ |
| 6 | 448208410 | T | C | stop_gained | +++ |
| 7 | 448208437 | T | C | stop_gained | +++ |
| 8 | 448208539 | T | C | stop_gained | +++ |
| 9 | 448208557 | T | C | stop_gained | +++ |
| 10 | 448208594 | A | G | stop_gained | +++ |
| 11 | 448208595 | A | G | stop_gained | +++ |
| 12 | 448208635 | T | C | stop_qained | +++ |
| 13 | 448208653 | T | C | stop_gained | +++ |
| 14 | 448208690 | A | G | stop_gained | +++ |
| 15 | 448208691 | A | G | stop_gained | +++ |
| 16 | 448208710 | T | C | stop_gained | +++ |
| 17 | 448208731 | T | C | stop_gained | +++ |
| 18 | 448208746 | T | C | stop_gained | +++ |
| 19 | 448208776 | T | C | stop_gained | +++ |
| 20 | 448208785 | T | C | stop_gained | +++ |
| 21 | 448208791 | T | C | stop_gained | +++ |
| 22 | 448208809 | T | C | stop_gained | +++ |
| 23 | 448208821 | T | C | stop_gained | +++ |
| 24 | 448208840 | A | G | stop_gained | +++ |
| 25 | 448208841 | A | G | stop_gained | +++ |
| 26 | 448208863 | T | C | stop_gained | +++ |
| 27 | 448208879 | A | G | stop_gained | +++ |
| 28 | 448208880 | A | G | stop_gained | +++ |
| 29 | 448208890 | T | C | stop_gained | +++ |
| 30 | 448208965 | T | C | stop_gained | +++ |
| 31 | 448209004 | T | C | stop_qained | +++ |
| 32 | 448209010 | T | C | stop_gained | +++ |
| 33 | 448209088 | T | C | stop_gained | +++ |
| 34 | 448209127 | T | C | stop_gained | +++ |
| 35 | 448209181 | T | C | stop_gained | +++ |
| 36 | 448209221 | A | G | splice_donor_variant | +++ |
| 37 | 448209372 | A | G | splice_acceptor_varian t | +++ |
| 38 | 448209480 | T | C | stop_gained | +++ |
| 39 | 448209549 | A | G | splice_donor_variant | +++ |
| 40 | 448209667 | A | G | splice_acceptor_varian t | ++ |
| 41 | 448209695 | T | C | stop_gained | ++ |
| 42 | 448209704 | T | C | stop_gained | ++ |
| 43 | 448209743 | A | G | splice_donor_variant | ++ |
| 44 | 448209816 | A | G | splice_acceptor_varian t | + |
| 45 | 448209856 | T | C | stop_gained | + |
| 46 | 448209880 | T | C | stop_gained | + |
| 47 | 448209883 | T | C | stop_gained | + |
| 48 | 448209925 | T | C | stop_gained | + |
| 49 | 448210075 | T | C | stop_gained | + |
| 50 | 448210081 | T | C | stop_gained | + |
| 51 | 448210123 | A | G | splice_donor_variant | + |
| 52 | 448210210 | A | G | splice_acceptor_varian t | + |
| 53 | 448210310 | T | C | stop_gained | + |
| 54 | 448210313 | T | C | stop_gained | + |
| 55 | 448210352 | T | C | stop_gained | + |
| 56 | 448210494 | A | G | splice_donor_variant | + |
| 57 | 448210590 | A | G | splice_acceptor_varian t | + |
| 58 | 448210614 | T | C | stop_gained | + |
| 59 | 448210659 | T | C | stop_gained | + |
| 60 | 448210686 | T | C | stop_gained | + |
| 61 | 448210692 | T | C | stop_gained | + |
| 62 | 448210722 | T | C | stop_gained | + |
| 63 | 448210731 | T | C | stop_gained | + |
| 64 | 448210740 | T | C | stop_gained | + |

**Table 2**

| SEQ ID NO: | Position KE_ZW6_hq-v1.chr6 Psat06G0558200 | Reference | Modification | Effect (genomic level) | Effect (protein level) |
|---|---|---|---|---|---|
| 67 | 448252681 | A | G | stop_gained | + |
| 68 | 448252699 | A | G | stop_gained | + |
| 69 | 448252729 | A | G | stop_gained | + |
| 70 | 448252735 | A | G | stop_gained | + |
| 71 | 448252762 | A | G | stop_gained | + |
| 72 | 448252807 | A | G | stop_gained | + |
| 73 | 448252831 | T | C | splice_acceptor_varian t | + |
| 74 | 448252905 | T | C | splice_donor_variant | + |
| 75 | 448252924 | A | G | stop_gained | + |
| 76 | 448253047 | A | G | stop_gained | + |
| 77 | 448253086 | A | G | stop_gained | + |
| 78 | 448253089 | A | G | stop_gained | + |
| 79 | 448253189 | T | C | splice_acceptor_varian t | + |
| 80 | 448253276 | T | C | splice_donor_variant | + |
| 81 | 448253318 | A | G | stop_gained | + |
| 82 | 448253324 | A | G | stop_gained | + |
| 83 | 448253474 | A | G | stop_gained | + |
| 84 | 448253516 | A | G | stop_gained | + |
| 85 | 448253519 | A | G | stop_gained | + |
| 86 | 448253543 | A | G | stop_gained | + |
| 87 | 448253546 | A | G | stop_gained | + |
| 88 | 448253561 | A | G | stop_gained | + |
| 89 | 448253601 | T | C | splice_acceptor_varian t | ++ |
| 90 | 448253702 | T | C | splice_donor_variant | ++ |
| 91 | 448253741 | A | G | stop_gained | ++ |
| 92 | 448253750 | A | G | stop_gained | ++ |
| 93 | 448253778 | T | C | splice_acceptor_varian t | ++ |
| 94 | 448253880 | T | C | splice_donor_variant | ++ |
| 95 | 448253949 | A | G | stop_gained | +++ |
| 96 | 448254057 | T | C | splice_acceptor_varian t | +++ |
| 97 | 448254207 | T | C | splice_donor_variant | +++ |
| 98 | 448254247 | A | G | stop_gained | +++ |
| 99 | 448254301 | A | G | stop_gained | +++ |
| 100 | 448254340 | A | G | stop_gained | +++ |
| 101 | 448254418 | A | G | stop_gained | +++ |
| 102 | 448254424 | A | G | stop_gained | +++ |
| 103 | 448254463 | A | G | stop_gained | +++ |
| 104 | 448254538 | A | G | stop_gained | +++ |
| 105 | 448254548 | T | C | stop_gained | +++ |
| 106 | 448254549 | T | C | stop_gained | +++ |
| 107 | 448254565 | A | G | stop_gained | +++ |
| 108 | 448254587 | T | C | stop_gained | +++ |
| 109 | 448254588 | T | C | stop_gained | +++ |
| 110 | 448254607 | A | G | stop_gained | +++ |
| 111 | 448254619 | A | G | stop_gained | +++ |
| 112 | 448254637 | A | G | stop_gained | +++ |
| 113 | 448254643 | A | G | stop_gained | +++ |
| 114 | 448254652 | A | G | stop_gained | +++ |
| 115 | 448254692 | T | C | stop_gained | +++ |
| 116 | 448254693 | T | C | stop_gained | +++ |
| 117 | 448254730 | A | G | stop_gained | +++ |
| 118 | 448254748 | A | G | stop_gained | +++ |
| 119 | 448254847 | A | G | stop_gained | +++ |
| 120 | 448254866 | T | C | start_lost | +++ |

As evident from the + to +++ ranking in the column Effect (protein level) in Tables 1 and 2, a ranking was performed showing the influence of a single signature mutation on level of expressed convicilin protein. An early stop codon causes a loss of transcription of RNA and thus a loss of translation to protein and thus, in sum, no transcription/translation at all. This leads to a reduced expression of convicilin and consequently to an increased gelling effect. The lower the expression the better is the effect on protein level.

| | |
|---|---|
| +++ | gelation reduced by 80 to 100% |
| ++ | gelation reduced by 40 to < 80% |
| + | gelation reduced by 0 to < 40% |

In certain embodiments, at least one signature mutation of Table 1 and of Table 2 will be combined. In certain embodiments, more than one mutation will be combined. In embodiments, where a high gelation is of interest, at least one signature mutation in each convicilin encoding gene / allele will be present, preferably each at least a ++, more preferably each a +++ mutation as detailed above.

The total pea protein ingredients produced by these selected pea lines or varieties have a lower convicilin content, or even no convicilin, and therefore significantly improved gelation, as compared to pea protein ingredients from plants in which the expression of one or more of the convicilin biosynthetic pathway genes is not reduced, significantly reduced, or eliminated. The selected pea plants are cultivated, and the peas may be harvested from these plants. The protein fraction can be extracted from the harvested peas, resulting in a pea protein ingredient having improved gelation properties as compared to a conventional pea protein ingredient made from peas in which the expression of one or more of the convicilin genes is not altered.

The method of the first aspect can, for instance, be used as part of or in combination with breeding programs, genome editing, mutagenesis, such as TILLING (Targeting Induced Local Lesions in Genomes) approaches, or other means of developing of new plant lines, including donor lines, comprising the modified convicilin alleles according to the present invention.

An allele can be detected by any means known in the art, including hybridization to allele-specific oligonucleotides, such as competitive allele specific PCR (KASP), or simply by nucleotide sequencing of the respective polymorphic region, including next generation sequencing and/or high throughput sequencing methods. Markers and suitable probes, including fluorescent probes, can be easily designed based on the mutation of the SNPs in the convicilin gene of particular interest as provided with the present invention.

In certain embodiments, the method may comprise an amplification step, such as PCR amplification or LCR (ligase chain reaction, a technique well known to the skilled person) amplification, to genotype one or more polymorphic positions and/one to detect one or more alleles associated with an improved trait DNA amplifications methods are well known in the art.

Embodiments relating to allele-specific amplification, including KASP, may comprise a non-allele-specific amplification step followed by allele-specific amplification or may use allele-specific amplification direction on the provided genomic DNA.

The method according to the present invention may be utilized for marker-assisted selection (MAS; Sun *et al.*, 2020), including genomic selection (GS) also referred to as genome wide selection (GWS).

Chemical- or radiation-induced mutagenesis is well established in the art and may be performed by any known suitable method, such as but not limited to EMS (ethyl methanesulfonate) mutagenesis or UV mutagenesis. As chemical- or radiation-induced mutagenesis is a random mutagenesis approach, expression of convicilin may be measured, as disclosed herein or by any suitable method known in the art. Through measuring the expression, mutated one or more plants having an increased expression of convicilin can be identified and/or selected after chemical- or radiation-induced mutagenesis.

Introduction may be through stable or transient introduction by means of transformation and/or insertion using genome-modification technology. Preferably, the introduction of the at least one functional copy comprised its integrated into the chromosomal plant genome and/or the chloroplast genome. Transformation may be performed by any method known in the art, such as but not limited to agrobacterium-mediated transformation, particle bombardment, PEG-mediated transformation, particle uptake or electroporation. Introduction through genome-modification technology may be performed by any site-specific genome modification technique known in the art using an SDN-3 approach, wherein the introduced sequence may be inserted by replacing at least on endogenous copy of a convicilin encoding gene or may be introduced elsewhere in the genome.

Embodiments using a) introduction and/or b) mutagenesis by genome modification, rely on a genome modification system, wherein a genome modification system refers to any DNA, RNA and/or amino acid sequence introduced into the cell, on a suitable vector and/or coated on a particles and/or directly introduced, wherein the genome modification system causes the modification of the genome of the cell in which it has been introduced, wherein the genome modification system comprises at least one site-directed nuclease, nickase or inactivated variant thereof, and optionally at least one further molecule, such as a guide molecule.

A "site-directed nuclease" herein refers to a nuclease or an active fragment thereof, which is capable of specifically recognizing and cleaving DNA at a certain location, the target sequence. Such nucleases typically produce a double-strand break (DSB), which is then repaired by non-homologous end-joining (NHEJ) or homologous recombination (HR). Site-specific nucleases include meganucleases, homing endonucleases, zinc finger nucleases, transcription activator-like nucleases and CRISPR nucleases, or variants including nickases or nuclease-dead variants thereof.

A "CRISPR nuclease", as used herein, is a specific form of a site-directed nuclease and refers to any nucleic acid guided nuclease which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. CRISPR nucleases also comprise mutants or catalytically active fragments or fusions of a naturally occurring CRISPR effector sequences, or the respective sequences encoding the same. A CRISPR nuclease may in particular also refer to a CRISPR nickase or even a nuclease-dead variant of a CRISPR polypeptide having endonucleolytic function in its natural environment. A variety of different CRISPR nucleases/systems and variants thereof are meanwhile known to the skilled person and include, inter alia, CRISPR/Cas systems, including CRISPR/Cas9 systems (EP2771468), CRISPR/Cpf1 systems (EP3009511B1), CRISPR/C2C2 systems, CRISPR/CasX systems, CRISPR/CasY systems, CRISPR/Cmr systems, CRISPR/MAD systems, including, for example, CRISPR/MAD7 systems (WO2018236548A1) and CRISPR/MAD2 systems, CRISPR/CasΦ systems (Pausch et al., Science, 2020, 10.1126/science.abb1400), CRISPR/CasZ systems and/or any combination, variant, or 30 catalytically active fragment thereof. A nuclease may be a DNAse and/or an RNAse, in particular taking into consideration that certain CRISPR effector nucleases have RNA cleavage activity alone, or in addition to the DNA cleavage activity.

The "guide molecule" or "guide nucleic acid sequence" (usually called and abbreviated as guide RNA, crRNA, crRNA+tracrRNA, gRNA, sgRNA, depending on the corresponding CRISPR system representing a prototypic nucleic acid-guided site-directed nuclease system), which recognizes a target sequence to be cut by the nuclease. The at least one "guide nucleic acid sequence" or "guide molecule" comprises a "scaffold region" and a "target region". The "scaffold region" is a sequence, to which the nucleic acid guided nuclease binds to form a targetable nuclease complex. The scaffold region may comprise direct repeats, which are recognized and processed by the nucleic acid guided nuclease to provide mature crRNA. A pegRNAs may comprise a further region within the guide molecule, the so-called "primer-binding site". The "target region" defines the complementarity to the target site, which is intended to be cleaved. A crRNA as used herein may thus be used interchangeably herein with the term guide RNA in case it unifies the effects of meanwhile well-established CRISPR nuclease guide RNA functionalities. Certain CRISPR nucleases, e.g., Cas9, may be used by providing two individual guide nucleic acid sequences in the form of a tracrRNA and a crRNA, which may be provided separately, or linked via covalent or non-covalent bonds/interactions. The guide RNA may also be a pegRNA of a Prime Editing system. The at least one guide molecule may be provided in the form of one coherent molecule, or the sequence encoding the same, or in the form of two individual molecules, e.g., crRNA and tracr RNA, or the sequences encoding the same.

In certain embodiments, a donor plant or donor plant population, comprising one or more alleles associated with an improved trait according to the present invention, may be crossed with a recipient plant or a recipient plant population, such as a plant of an elite line or any plant of interest, to introduce one or more alleles associated with an improved trait in the recipient plant or plant population, e.g. as part of a breeding program. The method according to the present invention may be used to identify one or more progenies of such crossings, having an improved trait.

In certain embodiments, one or more selected plants are used for one or more further breeding/crossing steps, including backcrossing, the one or more progenies of said one or more further crossing steps may be selected again for an improved trait by methods according to the invention. Cycles of crossing/breeding and selection of plants having an improved trait according to the present invention may be repeated multiple times.

The term "crossed" or "cross" refers to a sexual cross and involves the fusion of two haploid gametes via pollination to produce diploid progeny (e.g., cells, seeds or plants). The term encompasses both the pollination of one plant by another and selfing (or self-pollination, e.g., when the pollen and ovule are from the same plant).

"Backcrossing" refers to the process by which progeny are repeatedly crossed back to one of the parents, such as the (donor) parent comprising one or more alleles associated with an improved trait according to the present invention. In a backcrossing scheme, the "donor" parent refers to the parental plant with the desired gene/genes, locus/loci, or specific phenotype to be introgressed. The "recipient" parent (used one or more times) or "recurrent" parent (used two or more times) refers to the parental plant into which the gene or locus is being introgressed.

In certain embodiments, one or more plants having at least one improved trait according to the present disclosure, particularly an altered gelation pattern due to an decreased amount of convicilin expressed, may be identified directly, i.e. one or more parts of the plant, comprising genomic DNA of said one or more plants, is removed and used for the method according to the present invention, thereby identifying the presence of one or more alleles associated with an improved trait in, or representing the situation in, said one or more plants itself.

In certain embodiments, the method may comprise regeneration of an entire plant, preferably a fertile plant, from a plant cell, preferably derived from somatic tissue, embryonic tissue, callus tissue or protoplast. Regeneration may also be somatic embryogenesis, which is an artificial process in which a plant or embryo is derived from a single somatic cell or group of somatic cells. Somatic embryos are formed from plant cells that are not normally involved in the development of embryos, i.e. plant tissue like buds, leaves, shoots etc.

In certain embodiments, the method comprises the step of analyzing and/or verifying the desired trait phenotype of one or more selected plants as disclosed herein and/or by using any suitable method known in the art.

In a second aspect, there is provided a nucleic acid molecule comprising at least one modification of a nucleotide sequence of at least one gene, including the regulatory region thereof, the gene encoding a convicilin protein, wherein the modified nucleotide sequence is selected from any one of SEQ ID NOs: 3 to 64 or 1, having at least one modification in comparison to the reference gene sequence of SEQ ID NO: 2, and/or wherein the modified nucleotide sequence is selected from any one of SEQ ID NOs: 67 to 120, or 65 having at least one modification in comparison to the reference gene sequence of SEQ ID NO: 66, or any combination the aforementioned modifications of SEQ ID NOs: 3 to 64 and/or SEQ ID NOs: 67 to 120, or a nucleotide sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99% sequence identity to the respective sequence provided that the respective signature mutation as defined in Table 1 and Table 2 is still present, or a nucleic acid molecule comprising at least one modification of a nucleotide sequence of at least one gene, including the regulatory region thereof, the gene encoding a convicilin protein, wherein the modified nucleotide sequence comprises at least one signature mutation as defined in Table 1 and Table 2 in comparison to the reference gene sequence of SEQ ID NO: 2 or SEQ ID NO: 66, respectively.

In certain embodiments, the nucleic acid molecule is an isolated nucleic acid molecule.

In certain embodiments, there will be at least one signature mutation as defined in Table 1 and Table 2 in comparison to the reference gene sequence of SEQ ID NO: 2 or SEQ ID NO: 66, respectively in at least one convicilin gene of a *Pisum plant.* In another embodiments, there will be more than one signature mutation as defined in Table 1 and Table 2 in comparison to the reference gene sequence of SEQ ID NO: 2 or SEQ ID NO: 66, respectively in more than one convicilin allele of a *Pisum plant*, or even in all alleles. In yet another embodiment, more than one signature mutation as defined in Table 1 and Table 2 in comparison to the reference gene sequence of SEQ ID NO: 2 or SEQ ID NO: 66, respectively will be present in one and the same convicilin gene/allele of a *Pisum plant.*

In yet a third aspect, there is provided an expression construct or a vector comprising at least one modified nucleotide sequence of at least one convicilin gene and/or of the regulatory regions thereof of the second aspect. Embodiments of this third aspect are particularly suitable to introduce at least one SNP signature mutation from a plasmid of interest into a target cell for studying the effect thereof *in vitro* and *in vivo*, preferably by introducing the modification altering the gelation pattern of a pea protein ingredient in an inheritable way. In another embodiment, transient RNAi constructs may be constructed from the expression constructs or vectors to transiently modify the convicilin transcription in a cell or plant of interest.

In one embodiment of the third aspect, the present invention relates to an expression construct or vector, or a combination thereof, encoding a genome editing system, which targets at least one convicilin gene of a plant of the genus *Pisum,* or a functional or structural homologue thereof, wherein the genome editing system comprises (a) at least one site-specific nuclease or site-specific nickase, and optionally, in case a CRISPR system is used, at least one guide molecule or a sequence encoding the same, and (b) optionally, at least one repair template, or a sequence encoding the same.

In one embodiment of the expression construct or vector described above, the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasΦ system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof.

In an alternative aspect, the convicilin expression of one or more convicilin genes in a *Pisum plant can* be transiently reduced by a targeted knock-down of the transcription of the one or more convicilin genes to obtain a pea protein plant ingredient of the present invention without a modification of the plant on a genomic level. In one embodiment. In one embodiment of this knock-down method, an RNA silencing construct can be designed, introduced and used to reduce convicilin transcription, wherein said construct is, or the construct encodes an RNAi construct comprising a sense and an antisense sequence targeting a convicilin gene, or a functional or structural homolog thereof, the RNAi construct forming an RNA hairpin upon transcription. Suitable constructs for use in plant transformation (stable and transient) are known to the skilled person. Sense and antisense region(s) can be designed based on the sequence information provided herein.

The present inventors surprisingly observed that modifying the ratio of convicilin to vicilin storage proteins results in improved gelation properties, making the pea protein ingredient suitable for use in many applications. Such applications may include, but are not limited to, a food product, a beverage product precursor (e.g., highly viscous syrup), a nutraceutical product, a pharmaceutical product, a healthcare product, or a dietary supplement product.

In a fourth aspect, there is this provided a protein flour, a protein concentrate, a protein isolate, a pea protein texturate, a pea protein powder, or a pea protein flake of a *Pisum sativum* plant, having an altered gelation pattern, wherein the *Pisum sativum* plant comprises at least one modification of a nucleotide molecule in a nucleotide sequence of at least one convicilin gene, including the regulatory sequence thereof, wherein the nucleotide molecule is as defined in the second aspect above, and/or wherein the *Pisum sativum* plant, or a cell thereof, comprises at least one expression construct or at least one vector the third aspect above, or wherein the *Pisum sativum* plant comprises at least one modified convicilin protein as defined in the above first aspect, wherein the modification ist at least one signature mutation.

In a fifth aspect, there is provided a pea protein mixture or a pea protein composition comprising a protein flour, a protein concentrate, a protein isolate, a pea protein texturate, a pea protein powder, or a pea protein flake of a *Pisum sativum* plant of the forth aspect and additionally comprising at least one further additive and/or ingredient.

In certain embodiments, the pea protein ingredient, flour, protein concentrate, protein isolate, pea protein texturate, pea protein powder, or the pea protein flake of a *Pisum sativum* plant, having an altered gelation pattern, or any composition or mixture thereof, may be used as an alternative to animal-sourced proteins, resulting in a vegetarian or vegan option for consumers seeking meat or dairy alternatives. Examples of meat-alternative applications include burgers, sausages, and filets made with pea protein to replace some or all of the meat ingredients in such products. Examples of dairy-alternative applications include cheese-, yoghurt-, and milk-alternatives made with pea protein to replace some or all of the dairy ingredients in such products. Preferably, the pea protein composition having a high gelation pattern as disclosed herein will be particularly suitable for solid or semi-solid alternative food, or as basis for cosmetics (e.g., gels, crèmes and ointments and the like, usually having a semi-solid consistency to be easily applied), as the viscoelastic properties give structure, texture and stability to the product, which is otherwise hard to achieve with protein materials from natural sources.

The pea protein ingredient, flour, protein concentrate, protein isolate, pea protein texturate, pea protein powder, or the pea protein flake of a *Pisum sativum* plant, having an altered gelation pattern, produced by the selected pea plants preferably exhibits desired viscoelastic and other functional properties, particularly gelation, required for suitable extraction, processing, and production for use in consumable applications. In one embodiment, the desired properties are directed to the ability of the protein fraction to aggregate, i.e., form cross-linkages to form a stable gel mass that can be, for example, cut, sliced, shredded, or spread in a manner comparable to meat products or, preferably solid and semi-solid, dairy products. The meat products may include but are not limited to ground meat analogues, shredded meat analogues, and cut or fileted meat analogues. The dairy products may be hard, semi-hard, semi-soft, or soft cheese products, or spoonable or pourable dairy products, also including ice cream and the like.

In certain embodiments, a consumable product, preferably an alternative food product or an alternative cosmetic product, or a hybrid product, as described herein may include one or more lipid composition(s), for example a fat, an oil, or combinations thereof. In general, fats refer to lipid compositions that are solid at room temperature, whereas oils are liquid at room temperature. The lipid compositions may include saturated fatty acids (also referred to as "saturated fats"), unsaturated fatty acids (also referred to as "unsaturated fats"), or combinations thereof. The lipid composition may include, but are not limited to, vegetable oil, coconut oil, palm oil, sunflower oil, soy oil, canola oil, or combinations thereof. The consumable product may include between 1% and 80%, between 1% and 70%, between 1% and 10%, between 1% and 5%, between 5% and 30%, between 10% and 25%, between 10% and 75%, or between 15% and 70% by weight of a lipid composition depending on the type of dairy substitute. An ordinarily skilled artisan will understand the appropriate lipid composition inclusion rate for a given composition.

In certain embodiments, a cosmetic product or composition may additionally include approved water-soluble and/or oil-soluble UV-A, UV-B and / or Broadband filter substances and optionally further additives or ingredients used in cosmetics, for example perfume, dyes, antimicrobials, lipid-replenishing agents, complexing and sequestering agents, pearlescent agents, other plant extracts, vitamins, active ingredients, preservatives, bactericides, repellents, self-tanners, depigmenting agents, pigments that have a coloring effect, softening, moisturizing and / or moisturizing substances, or other usual components of a cosmetic or dermatological formulation such as emulsifiers, polymers, foam stabilizers and electrolytes. Preferably, the protein flour, a protein concentrate, a protein isolate, a pea protein texturate, a pea protein powder, or a pea protein flake of a *Pisum sativum* plant, having an altered gelation pattern is used as a natural stabilizer alternative.

In certain embodiments, the pea protein ingredient, flour, protein concentrate, protein isolate, pea protein texturate, pea protein powder, or the pea protein flake of a *Pisum sativum* plant, having an altered gelation pattern, or any composition or mixture thereof, may thus be favorably used as cosmetic thickener or stabilizer, as the natural pea-derived product with increased gelation helps to increase viscosity in cosmetic water and water-in-oil based emulsions or to favorably influence the rheology of a cosmetic product or composition.

The alternative food product, or the alternative cosmetic product, or a hybrid product, may include water as needed. For example, the product may include between 1% and 80%, between 5% and 75%, between 15% and 70%, between 45% and 65%, between 50% and 60%, between 1% and 20%, or between 5% and 15% by weight of water depending on the type of consumable product.

In some embodiments, the consumable product comprising a pea protein ingredient, flour, protein concentrate, protein isolate, pea protein texturate, pea protein powder, or a pea protein flake of a *Pisum sativum* plant, having an altered gelation pattern includes both water and a plant-based ingredient. The total of the water and plant-based ingredient may be e.g. between 50% and 95% or between 60% and 90% by weight of the composition.

In certain embodiments, the consumable product comprising a pea protein ingredient, flour, protein concentrate, protein isolate, pea protein texturate, pea protein powder, or a pea protein flake of a *Pisum sativum* plant, having an altered gelation pattern may include fiber. The fiber may include, but is not limited to, pectin, apple fiber, psyllium, flax fiber, rice bran extract, Konjac flour, and the like. The consumable product may include between 0.01% (wt) and 3% (wt), between 0.05% (wt) and 2% (wt), or between 0.1% (wt) and 2% (wt) of fiber. The consumable product may include fiber in an amount up to 0.5% (wt), up to 1% (wt), up to 1.5% (wt), up to 2% (wt), up to 2.5% (wt), or up to 3% (wt).

In certain embodiments, the consumable product comprising a pea protein ingredient, flour, protein concentrate, protein isolate, pea protein texturate, pea protein powder, or a pea protein flake of a *Pisum sativum* plant, having an altered gelation pattern may include starch. The starch may include a pregelatinized starch, a modified starch, or combinations thereof. The starch may include, but is not limited to, com starch, potato starch, tapioca starch, and the like. The consumable product may include between 0.5% (wt) and 25% (wt), between 1.0% (wt) and 20% (wt), or between 2% (wt) and 18% (wt) of starch. The consumable product may include a hydrocolloid. For example, the consumable product may include guar gum, xanthan gum, locust bean gum, carrageenan, cellulose, konjac gum, and combinations thereof. The consumable product may include between 0.01% and 5%, between 0.05% and 4.5%, between 0.1% and 4.0%, or between 0.5% and 3.8% by weight of hydrocolloid. The consumable product may include up to 5%, up to 4.5%, up to 4.0%, up to 3.8%, up to 3.5%, up to 2.5%, up to 2.0%, or up to 1.0% by weight of hydrocolloid.

In some embodiments, the consumable product comprising a pea protein ingredient, flour, protein concentrate, protein isolate, pea protein texturate, pea protein powder, or a pea protein flake of a *Pisum sativum* plant, having an altered gelation pattern may include lecithin. The consumable product may include between 0.01% and 10%, between 0.05% and 8.0%, or between 0.1% and 5% by weight lecithin.

In certain embodiments, the consumable product comprising a pea protein ingredient, flour, protein concentrate, protein isolate, pea protein texturate, pea protein powder, or a pea protein flake of a *Pisum sativum* plant, having an altered gelation pattern may include a preservative. For example, the consumable product may include a preservative such as, but not limited to potassium sorbate. The consumable product may include a preservative in an amount up to 0.1%, up to 0.5%, or up to 1.0% by weight of the consumable product. The consumable product may include a flavour or seasoning. For example, the consumable product may include a natural or artificial flavour(s) and/or seasonings. Seasonings may include, but are not limited to, sweetener(s), salt (e.g., sodium chloride, potassium chloride, and the like), cocoa, chocolate, cinnamon, nutmeg, coconut, almond, combinations thereof, and the like. The consumable product may include between 1% and 20%, between 1.5% and 10%, between 5% and 20%, or between 2% and 18% of a flavour or seasoning. The consumable product may be free of any flavours or seasoning. In some embodiments, the consumable product may include between 0.001% and 3.0%, between 0.01% and 2.0%, or between 0.025% and 1.75% of a salt. The consumable product may be free of salt.

In yet another embodiment, the consumable product comprising a pea protein ingredient, flour, protein concentrate, protein isolate, pea protein texturate, pea protein powder, or a pea protein flake of a *Pisum sativum* plant, having an altered gelation pattern may include a sweetener. Suitable sweeteners are known and described in the art. The sweetener can be at least one of a non-caloric sweetener or a caloric sweetener. The sweetener can be any type of sweetener, for example, a sweetener obtained from a plant or plant product, or a physically or chemically modified sweetener obtained from a plant, or a synthetic sweetener. Exemplary sweeteners include steviol glycosides, mogrosides, sucrose, fructose, glucose, erythritol, maltitol, lactitol, sorbitol, mannitol, xylitol, tagatose, trehalose, galactose, rhamnose, cyclodextrin (e.g., a-cyclodextrin, b-cyclodextrin, and g-cyclodextrin), ribulose, threose, arabinose, xylose, lyxose, allose, altrose, mannose, idose, lactose, maltose, invert sugar, isotrehalose, neotrehalose, palatinose or isomaltulose, erythrose, deoxyribose, gulose, idose, talose, erythrulose, xylulose, psicose, turanose, cellobiose, glucosamine, mannosamine, fucose, fuculose, glucuronic acid, gluconic acid, glucono-lactone, abequose, galactosamine, xylo-obgosaccharides (xylotriose, xylobiose and the like), gentio- obgoscaccharides (gentiobiose, gentiotriose, gentiotetraose and the like), galacto- obgosaccharides, sorbose, ketotriose (dehydroxyacetone), aldotriose (glyceraldehyde), nigero- obgosaccharides, fructoobgosaccharides (kestose, nystose and the like), maltotetraose, maltotriol, tetrasaccharides, mannan-oligosaccharides, maltooligosaccharides (maltotriose, maltotetraose, maltopentaose, maltohexaose, maltoheptaose and the like), dextrins, lactulose, melibiose, raffmose, rhamnose, ribose, sucralose, acesulfame K, aspartame, saccharin, coupling sugars, soybean oligosaccharides, and combinations thereof. D- or L-configurations can be used when applicable.

The consumable product comprising a pea protein ingredient, flour, protein concentrate, protein isolate, pea protein texturate, pea protein powder, or a pea protein flake of a *Pisum sativum* plant, having an altered gelation pattern may include an acid. Suitable acids include, but are not limited to, citric acid, lactic acid, sorbic acid, malic acid, combinations thereof, and the like. The consumable product may include an acid in an amount up to 0.001%, up to 0.005%, up to 0.01%, up to 0.1%, up to 1.0%, up to 1.5%, or up to 2.0% of the consumable product. The consumable product may include between 0.0001% and 2.0%, between .0002% and 1.5%, between 0.0003% and 1.0% by weight of an acid.

According to certain embodiments disclosed herein, the alternative product is a hybrid product that comprises a pea protein ingredient of the present invention as well as at least further additive and/or ingredient. Besides additives and ingredients typically used for food and cosmetic production, also at least one lab-grown material can be used. A lab-grown material as used herein refers to a cultured and/or fermented cell, and the material (any biomass, cellular and non-cellular, including supernantant) obtained therefrom, including bacterial cells, fungal cells, including filamentous fungi, particularly fungi and fungi biomass suitable as meat substitute, and and cultured animal cells, including hepatocytes, myoblasts, osteoblasts, fibroblasts, lipoblasts, odontoblasts, adult neuronal progenitor cells, neural stem cells, multipotent stem cells from subventricular forebrain region, ependymal-derived neural stem cells, hematopoietic stem cells, liver-derived hematopoietic stem, marrow-derived stem cell, adipo-fibroblasts, adipose-derived stem cells, islet- cells producing stem cells, pancreatic-derived pluripotent islet-producing stem cells, mesenchymal stem cells, placenta cells, bone marrow stromal cells, muscle side population cells, bone marrow-derived recycling cells, blood-derived mesenchymal precursor cells, bone- marrow derived side population cells, muscle precursor cells, circulating skeleton stem cells, neural progenitor cells, multipotent adult progenitor cells, mesodermal progenitor cells, spinal cord progenitor cells and spore-like cell, and any combinations thereof, wherein the cell is not derived from a human embryo.

In certain embodiments, a hybrid food or composition may thus comprise at least one portion being a pea protein ingredient, flour, protein concentrate, protein isolate, pea protein texturate, pea protein powder, or a pea protein flake of a *Pisum sativum* plant of the present invention and at least one portion being a lab-grown material, preferably wherein the lab-grown material stems from a bacterial fermentation, or from a fungus, preferably from a filamentous fungus. Filamentous fungal biomass, usually named mycoproteins, is a suitable meat substitute since it is nutritious and has filaments and thus a texture perfectly mimicking meat fibrils. Regarding the type of residual water, nutrient supplementation, optimum conditions for biomass production, and characteristics of the mycoproteins, the optimum growth condition can be at about pH of 4.5. Mycoprotein usually contains 19.44% (*wt.*/*wt.*) protein with a high crude fiber content of 8.51% (*wt.*/*wt.*) and a low fat content of 1.56% (w/w). In addition, the amino acid and fatty acid contents are dominated by glutamic acid and polyunsaturated fatty acids, which are associated with an umami taste (Wikandari et al., 2023, https://doi.org/10.3390/molecules28030997). Particularly the physico-chemical and the viscoelastic properties of mycoproteins and the pea protein ingredients of the present invention allow that both sources of alternative proteins are easily compounded and used together for food, particularly alternative meat or fish design, as the pl values of the major protein fractions and the viscoelastic properties are perfectly suitable to produce food with a high nutrition value and an excellent texture and stability.

In certain embodiments, depending on the nature of the ultimate product of interest comprising a pea protein ingredient, flour, protein concentrate, protein isolate, pea protein texturate, pea protein powder, or a pea protein flake of a *Pisum sativum* plant, having an altered gelation pattern of the present invention, the product may include further additives and ingredients. The skilled person being a food chemist, expert, a chemist, a cosmetics engineer or a process engineer will well be in a position to adjust the final content of a product to achieve the optimum conditions guaranteeing easy and efficient manufacturing as well as a hygienically safe, food- or cosmetics-grade product with the right degree of stability and non-perishable nature as needed.

In a sixth aspect, there is provided an alternative food or a cosmetic composition or product, or a hybrid composition or product, being selected from a solid and a semi-solid alternative food or cosmetic composition or product, comprising at least one protein flour, a protein concentrate, a protein isolate, a pea protein texturate, a pea protein powder, or a pea protein flake of a *Pisum sativum* plant claim of fourth aspect, or comprising at least one mixture of claim the fifth aspect, wherein the alternative food or an alternative cosmetic composition has an improved gelation in comparison to a reference material.

In one embodiment of the sixt aspect, the alternative food or the cosmetic composition or product, or the hybrid composition or product thereof has a pH in the range of about 3.5 to about 8.5, preferably a pH from about 4.5 to about 8.

In certain embodiments, particularly for alternative food or hybrid food production and applications, it is useful for the pea protein ingredient to be able to form a gel mass that can be sliced, shredded, crumbled, or spread. In view of the high gelation pattern of the pea protein ingredient, flour, protein concentrate, protein isolate, pea protein texturate, pea protein powder, or the pea protein flake of a *Pisum sativum* plant of the present invention, these proteins are particularly suitable for use in preparing a gel mass with a certain stability. The ability of the gel mass to function in these applications depends on the viscoelastic properties of the pea protein ingredient itself, and/or in combination with additional ingredients as needed for the desired consumable application. With the products and compositions presented herein, and using the signature mutations as presented above and the agar ranking and vicilin to convicilin ratio providing a link between genetics and gelation properties, alternative and hybrid products and compositions can be prepared using the pea protein ingredients having an altered vicilin to convicilin ratio to influence the stability and rheology of the desired products based on the natural pea protein ingredient as stabilizer and texturing agent.

In certain embodiments, to form the stable gel mass, the pea protein ingredient, optionally in combination with other ingredients to form a composition, is heated to the peak range of the DSC of the pea protein concentrate or the composition, preferably completely, as quickly as possible, followed by rapidly bringing the temperature down to below the DSC peak range as soon as possible after reaching the maximum peak temperature. It is particularly preferred to reach the peak end temperature of the denaturation peak of the composition to thus produce a stable gel mass with optimal properties.

Heating the pea protein ingredient or composition, generally to a temperature of around at least 100°C, depending on the peak temperature range, is preferably performed in a closable, pressure-tight vessel (pressure vessel) which is designed for a corresponding overpressure resulting from the steam released during heating. he absolute pressure in the vessel may be between 1.3 and 5 bar, preferably between 2 and 3 bar. Upon heating under the counterpressure, the stable gel mass is formed, having a storage modulus G' in a plate-plate rheometer of at least 30,000 Pa, preferably at least 50,000 Pa, more preferably between 50,000 Pa and 150,000 Pa, even more preferably between 50,000 Pa and 100,000 Pa.

In a seventh aspect, there is provided a method of producing a protein flour, a protein concentrate, a protein isolate, a pea protein texturate, a pea protein powder, or a pea protein flake of a *Pisum sativum* plant as defined in the fourth aspect above , or of a mixture or composition as defined in the fifth aspect above, comprising the steps of: (i) providing at least one pea from a *Pisum sativum* plant having an altered gelation pattern as defined in the first aspect above, or comprising at least one nucleotide molecule as defined in the second aspect above, or comprising at least one expression construct or vector as defined in the sixth aspect above; and (ii) optionally: dehulling the at least one pea; (iii) milling the at least one pea to obtain a flour; (iv) extracting at least one protein fraction, optionally: also including at least one starch fraction and/or at least one fat fraction, preferably wherein the extracting step is a dry extraction method, or wherein the extracting step is a wet extraction method; and (v) optionally: further processing and/or purifying the extracted protein fraction and thus obtaining an enriched and/or purified fraction selected from the group consisting of: protein flour, a protein concentrate, a protein isolate, a pea protein texturate, a pea protein powder, or a pea protein flake of a *Pisum sativum* plant.

In certain embodiments of the seventh aspect, whole peas, with, but pereferably without a step of dehulling, may be put into an aqueous composition for fermentation followed by milling the fermented peas, fractionating the fermented and milled peas so as to obtain at least one protein comprising fraction and (d) isolating and optionally purigying pea proteins from said at least one protein comprising fraction. Such a step of fermentation may be suitable to remove ono-, di-, and/or oligosaccharides and thus to enrich the protein fraction.

In an eigth aspect, there is provided a method of screening and/or selecting a *Pisum sativum* plant, wherein a pea protein ingredient obtained from said *Pisum* plant, preferably as intermediate, has an altered gelation pattern as defined in the first aspect, or wherein said plant comprises at least one nucleotide molecule as defined in the second aspect, wherein the method comprises: (i) using at least one probe or at least one primer capable of identifying a signature mutation of the present invention; (ii) identifying and optionally: obtaining at least one *Pisum sativum* plant having at least one modification of a nucleotide sequence of at least one gene, including the regulatory region thereof, the gene encoding a convicilin protein, as defined in the first aspect, or a combination of more than one modification.

In certain embodiments, suitable sets of PCR primers or probes, preferably labelled molecular probes, can be designed using the signature mutations as position markers to screen a germplasm for the presence or absence of at least one modification in a convicilin gene in comparison to a reference convicilin gene not carrying the relevant modification.

In a ninth aspect, there is provided a use of a protein flour, a protein concentrate, a protein isolate, a pea protein texturate, a pea protein powder, or a pea protein flake of a *Pisum sativum* plant as defined in the fourth aspect, or a use of a mixture as defined in the fifth aspect for preparing an alternative food composition or an alternative cosmetic composition, preferably wherein the food composition is a solid or semi-solid alternative food composition or an alternative cosmetic composition.

In certain embodiments, there is provided a protein flour, a protein concentrate, a protein isolate, a pea protein texturate, a pea protein powder, or a pea protein flake of a *Pisum sativum* plant or a mixture or composition thereof for use as ingredient in an alternative food or cosmetic product or composition, optionally being a hybrid product or composition, wherein the composition may be an oral cavity care composition, particularly a cream, gel, ointment, dental gel or cream and the like. In view of the viscoelastic properties of the protein compositions and isolates discloses herein, and further in view of the fact that pea convicilin and variants thereof as disclosed herein can be produced in a sustainable way and represent products as such known to be safe for human use, the gelation properties can be used for cosmetic and further care compositions of the human or animal body as alternative to chemical substances in view of the favorable physico-chemical properties of the convicilin-containing material disclosed herein and its inherent tolerability and safety.

While several possible aspects are disclosed above, embodiments of the present invention are not so limited. These exemplary aspects are not intended to be exhaustive or to unnecessarily limit the scope of the invention, but instead were chosen and described in order to explain the principles of the present invention so that others skilled in the art may practice the invention. Indeed, various modifications of the invention in addition to those described herein will become apparent to those skilled in the art from the foregoing description. Such modifications are intended to fall within the scope of the appended claims.

The present invention is, in particular, captured by any one or any combination of one or more of the above-mentioned aspects, with any other statement and/or embodiments.

The following Examples serve to provide further details on embodiments and enablement of the invention, but do not necessarily limit the scope of the invention.

### Example 1: Bioinformatic analysis and selection and screening for suitable lines

To verify the hypothesis that a targeted reduction of convicilin can indeed influence the gelation properties of pea protein ingredients in an adjustable manner, several in house lines and germplasm were analyzed and compared to sequences *Pisum* genomes of Cameor, further in-house lines / loci / germplasm, and genome assembly data, e.g. for ZM6, were analyzed in detail (cf. Kreplak et al., 2019, and Yang et al., 2022, both *supra*)*.*

Based on *in silico* predictions, the impact of the different types of pea storage proteins on gelation behaviour was assessed in a targeted way using a complex bioinformatic and biochemical analysis of various pea lines based on prior studies, and the following findings of the inventors were made: ~ twenty pea lines were selected from the breeding collection, representing the greatest haplotype diversity in protein storage genes. From these lines, those which were able to produce a preferred pea protein are selected for further development. Selection criteria include but are not limited to pea plants producing peas with a lower convicilin content, a lower albumin content, or a combination of both properties.

Specifically, based on a detailed annotation, comparison and targeted mutagenesis of the pea reference genome data available, specific variants could be identified that altering the expression of a convicilin gene, such as by mutation, deletion, or suppression of expression, it is possible to modulate the convicilin content of peas harvested from the cultivated plant. The levels of other storage proteins in peas may also be modulated by a similar method.

Notably, as summarized in Tables 1 and 2 above, specific SNP mutations and certain mutations called "signature mutations" were identified that contribute either to a loss of transcription, or a loss of translation of functional convicilin copies (usually two in a pea genome).

Based on these findings, and particularly the SNPs identified, TILLING populations can be screened with the help of suitable primers and markers to identify the "convicilin low / high gelation +++" germplasm, especially that group having an almost abolished convicilin production, can be easily identified. Further, targeted gene editing can be used, e.g., using CRISPR/Cas technologies, by designing suitable gRNAs targeting the relevant convicilin gene regions (coding / non-coding / upstream and downstream regulatory regions) to provide pea germplasm producing total protein ingredients that have excellent gelation properties, which is usually a highly limiting factor for standard *Pisum sativum* pea protein preparations, making pea as valuable plant an even more interesting source for plant-based protein production.

### Example 2: Production of pea flour from whole peas

The material used was as follows: whole peas, any suitable variety, several kilograms; several bowls of adequate size for sorting; scoops and a brush; a Streckel&Schrader laboratory dehuller LU-200; a Streckel&Schrader laboratory airleg separator LST1 and a ultracentrifugal mill Retsch ZM200 with cyclone, attached bowl and 0,2mm sieve.

To obtain a pea protein flour, the following sample preparation was conducted: for the dehulling step, the whole peas were put into the dosing funnel of the dehuller. Using the dosing channel, the speed and amount of peas falling into the dehulling chamber can be adjusted. The slit between the dehuller discs can be adjusted to the size of the peas. The mix of dehulled whole and split peas and their hulls is collected in a drawer below the discs and is transferred into the dosing funnel of the airleg separator in order to sort them apart. The air flow to the different chambers therein can be adjusted with two screws. Peas that are still not dehulled, are transferred back to the dehullerwhile adjusting the slit accordingly.

Dehulled peas were then milled with the ultracentrifugal mill and the mentioned components, carefully dosing them with a scoop. The resulting flour is collected in a bowl, and residues on parts of the mill are brushed off in order to clean them.

### Example 3: Extraction of Pea Protein Isolate (PPI) from pea flour

To this end, the following material was used: pea flour (e.g., as produced in Example 2 above; mixtures of flour from different peas or even of different plants can be used), 1M NaOH, 6M HCl, distilled water, 3L and 5L beakers, a lab scale PCE Instruments PCE-BSH 10000, a propeller stirrer VWR VOS 40 digital, a magnetic stirrer IKA RH 2 basic, a centrifuge Eppendorf 5910 Ri cooled, with 4 centrifuge bottles 1L, a dispersed homogenizer IKA ultraturrax T50 digital with S 50 N G45G disperser tool, a pH meter Mettler Toledo FiveEasy F20, a spoon, silicone spatula and metal spatula, and a spray dryer Büchi S-300.

Pea flour was dispersed into distilled water while stirring with a propeller stirrer at around 400 *rpm* in a ratio of S:L of 1:7, e.g. 500 *g* flour and 3000 *mL* water. The pH value of the dispersion was measured and adjusted using a pH-meter and 1M NaOH until it reached pH 8. Extraction was conducted at 150 *rpm* at room temperature (at about 20 to 22°C) for 1 h. The dispersion was then transferred into the centrifugation bottles and centrifuged at 4347 × g for 25 *min* to separate the dissolved protein-rich supernatant from the pellet rich in starch and fibers. The pellet was disposed. The supernatant was transferred to the 3L beaker and stirred with the magnetic stirrer in order at 500 *rpm* to carefully dose the 6M HCL to precipitate the protein. The pH was adjusted to the isoelectric point of the proteins at 4.5, and the protein dispersion was stirred for 10 *min* to help the proteins agglomerate better. The dispersion was then centrifuged at 4347 × g for 25 *min* at 7 °*C* to lower the protein solubility further and improve the protein yield. The resulting supernatant was disposed, and the protein pellet was carefully removed from the bottles with spatulas and transferred into a beaker. The protein was washed with distilled water at a ratio of S:L of 1:5, using a ultraturrax disperser. The washed protein was again centrifuged under aforementioned conditions, and collected in a beaker. Neutralisation was carried out using the ultraturrax homogeniser, distilled water, until a protein concentration of about 10 % was reached, and 1M NaOH. The neutralized protein solution was then spray-dried under the following conditions: inlet temperature 130 °*C*, spraying gas flow 900 *L*/*h*, drying gas flow 35 *m³,* product flow 6 *mL*/*min.* With these adjustable parameters the resulting outlet temperature was kept between 70-75 °C to ensure a safe product while maintaining the functionalities of native proteins as much as possible.

Specific parameters of this process are not intended to be limiting, and variations of this extraction process by adjusting the pH, temperature and standing time, and other parameters may be utilized.

The standardized workflow from pea flour to pea isolate is shown in **Figure 1****.** **Figure 2 (Fig. 2A to E)** shows some working steps during the extraction process.

**Figure 3** provides a detailed flow scheme for the Pea Protein Isolate (PPI) - Extraction Method: AE-IEP (Alkaline Extraction with Isoelectric Precipitation) as established. For this method, extraction at RT with a propeller stirrer is performed at 150 rpm, followed by protein precipitation on a magnetic stirrer at 400 rpm. This is followed by washing and neutralization, next by resuspension and homogenisation in distilled water with IKA ultra turrax T50. Finally, spray drying with Büchi S-300 is performed. Parameters: Inlet-Temp. 130°C; Outlet-Temp. 70-75°C; Product flow: 6 mL / min; spraying gas: 900 L/h, drying gas: 35m³/h. To this end, a stable PPI can be obtained.

### Example 4: Protocol for sample preparation and storage protein ratio determination

Ratio determination in all pea products was performed by CE-SDS (capillary electrophoresis with SDS buffer).

The material used was an Agilent ProteoAnalyzer, an Agilent Protein Broad Range P240 Kit, a Vortex IKA VXR basic Vibrax, a centrifuge Heraeus Biofuge pico, a Pipette set (Eppendorf research 2-20 µl, 100-1,000 µl), a sodium dodecyl sulfate (SDS, Thermo Scientific) PAGE done under reducing conditions using dithiothreitol (DTT) Serva.

Sample preparation was then performed as follows: for each experiment, 10 mg of each ground sample was mixed with 1 ml of a 1% SDS solution containing 0,4% dithiothreitol. The mixture was vortexed for 30 minutes to ensure thorough mixing and lysis of the sample. After vortexing the mixture was centrifuged at 15.000 g for 5 minutes to remove large particles and unbroken cells. Carefully, 4 µl of the supernatant was collected.

Sample analysis was subsequently performed by using the obtained supernatant, containing the solubilized proteins, which was prepared and analyzed using microfluidic gel electrophoresis according to the manufacture's assay guide. The electropherogram peaks were assigned to the corresponding protein fragments based on their molecular weight: gamma-vicilin (14-16 kDa), alpha-vicilin (19-20 kDa), basic legumin (22 kDa), beta+gamma-vicilin (25-30 kDa), alpha+beta-vicilin (36 kDa), acidic legumin (38-40 kDa), vicilin (48-51 kDa), convicilin (~75 kDa), and lipoxygenase (90-95 kDa).

Comparative measurements and visualization and quantification are shown in **Fig. 4** and **Fig. 5****,** taken from the line **KWS 1 (****Fig. 4****),** for line **KWS 2 (****Fig. 5A to 5C****),** and for Proklam **(****Fig. 5D to 5F****),** all further detailed in **Example 5** below. For line KWS 1 having a signature mutation, a significantly higher vicilin to convicilin ratio was identified. In contrast, the ratio was lower for KWS 2 and for Proklam not carrying the signature mutation as detailed above. For Proklam, a total vicilin to convicilin ratio of 2.7:1 was identified.

Notably, KWS 1 was shown to carry signature mutations as shown in **Tables 1** and **2** leading to a favorably altered gelation pattern as characterized by a total vicilin to convicilin ration of ~ 5:1 that is much higher than the standard ratio of 2:1 to 4:1 defined as the standard ration present in a variety of pea lines (as average) not showing one of the signature mutations of **Table 1** and/or **Table 2** in its genome. Interestingly, as for many pea lines, it was confirmed that the average standard ratio of vicilin to convicilin is usually between about 2:1 to about 4:1 (wt./wt.). This value was confirmed over a variety of reference lines tested (average of about 100 lines and varieties available and screened). For our studies, lines were specifically screened for and/or generated that have a lower amount of convicilin, i.e., a ration shifted to vicilin until a complete knock-out of detectable convicilin. These convicilin-low lines indeed showed a significantly enhanced gelation correlated to the decreased amount of convicilin present in the samples tested. Ground flour was used as reference as it represents the almost unprocessed pea protein containing sample after dehulling, yet it was also confirmed that the ratio of vicilin to convicilin was also detectable in a comparable ratio in further processed (PPI) samples (data not shown).

As further detailed below, the easy read-out parameterof a vicilin to convicilin ratio perfectly matched with a further test established, an agar ranking test (see **Example 5).** With these both tests, an quick screening on "gelation-high" lines can be performed and the association with a signature mutation in a convicilin gene of the present invention can be further tested and screened for.

### Example 5: Gelling Test

To establish a standardized test, the following appliances were used: a Vorwerk THERMOMIX TM6 with steamer attachment; a Microwave sharp inverter, a 100 mL Cups (e.g., Sarstedt), petri dishes (glass, diameter 6 cm); a silicone spatula, a metal spatula, Agar (Invitrogen, Select Agar, qualified for molecular genetics applications), a protein powder (protein powder can be used as a convenient and standardized equivalent in the test established for all protein extracts, isolates and the like, in the present experiment a protein isolate with a protein content of 85% was used).

Sample preparation for the gelation test was then performed as follows: 4 g protein powder was stirred into 16 g water in a 100 mL Sarstedt Cup using a silicone spatula. Another set of samples were prepared for each protein powder with a dilution of 5 g protein powder and 15 g water. Stirring was carried out until a homogeneous mass was obtained. The samples were then left to soak for one hour at room temperature (about 20°C to about 22°C). During this time, the samples were thoroughly mixed after 30 min and again after 60 min.

Next, a steam treatment was performed. After the soaking phase, the samples were steamed using a Vorwerk Thermomix TM6. For this purpose, the samples were transferred to glass Petri dishes and placed in the steam cooking attachment of the appliance. Steaming was carried out for 35 min at 95°C. The samples were then cooled on ice for 15 min.

Subsequently, the following steps were carried out for sampling and analysis: 1. photo documentation: the samples were photographed to record visual changes. 2. spatula sampling: A sample was taken with a spatula to check the consistency and homogeneity. 3. spatula scratch sample: Another sample was taken by scratching with a spatula to assess the texture. 4. Documentation of optical attributes: The optical attributes of the samples were recorded in detail. 5. agar equivalence test: Standards of 0; 1; 1.1; 1.2; 1.3; 1.4; 1.5; 1.6; 1.7 and 1.8 g/L were prepared from agar equivalent to the protein powder sample preparation. This dilution series was used to cluster the samples from 1 to 10 to be tested against a uniform standard. The agar standard of 0 g/L corresponds to a value of 1, the agar standard of 1 g/L corresponds to a value of 2, the agar standard of 1.1 g/L corresponds to a value of 3, the agar standard of 1.2 g/L corresponds to a value of 4, the agar standard of 1.3 g/L corresponds to a value of 5, the agar standard of 1.4 g/L corresponds to a value of 6, the agar standard of 1.5 g/L corresponds to a value of 7, the agar standard of 1.6 g/L corresponds to a value of 8, the agar standard of 1.7 g/L corresponds to a value of 9, the agar standard of 1.8 g/L corresponds to a value of 10.

The results of the gelation and the associated optical and textural changes were carefully recorded and analyzed.

**Table 3**

| | **KWS 1** | **KWS 2** | **KWS 3** | **Proklam (Commercially available)** |
|---|---|---|---|---|
| Drying process | spray-dried | spray-dried | spray-dried | spray-dried |
| Color of the gel | Olive color | Bright light vellow | Bright light vellow | Dark yellow, reddish |
| Foam building | The bubbles have spread throughout the gel. | The bubbles have spread throughout the gel. | The bubbles have spread throughout the gel. | Lass Foam, than the other gels, but also spread throughout the gel |
| Gelling test 25 % | solid gel KWS 1,2,3 are similar | solid gel | solid gel | Moderately solid gel |
| Stitch test 25 % | Puncture site recognizable, puncture-proof | Puncture site flows shut again, not puncture-proof | Puncture site recognizable, puncture-proof | Not puncture resistant, elastically deformable, liquid part |
| Scratch test 25 % | Clear boundary, similar to KWS 4 | Flowing back together | Clear border Most scratch-resistant | Flowing back together, watery |
| Scratch test 25 % observation after 24 hours | Bottom is clear, Scratch edges are pretty straight | Scratch hole is closing a little, Fluid is leaking on the bottom of the scratch hole | Bottom is clear, Scratch edges are falling a little bit apart | Flowing back together, watery |
| Agar ranking | 8 | 4 | 7 | 2 |

The results for the direct measurement and for the measurement after 24 h are shown in **Figure 6 (Fig. 6A** to **Fig. 6H****).** By establishing the agar ranking test, a reliable and standardized test could be established that allows to classify pea material according to its gelation pattern. Whereas KWS Proklam, for example, provides a rather liquid formulation with a low gelation pattern, the different KWS lines KWS 1 to KWS 3, all showed a higher gelation pattern, with KWS 3 as established and identified and carrying signature mutation having the strongest gelation of the compared gels.

Gel stability was positively correlated with a decrease of convicilin expressed. To this end, in addition to the total storage protein measurement allowing the determination of a favorable high gelation pattern (meaning a reduced amount of convicilin and thus an increased amount of the ratio vicilin to convicilin), an easy agar ranking was established that provides a visual read-out perfectly correlating with the amount of vicilin / convicilin identified on a molecular scale. As shown in Table 3 above, a scale from 1 to 10 was established, 1 meaning almost liquid and 10 meaning very firm. Interestingly, the visually rather liquid (Proklam, Agar ranking 2) and not too stable in the test setting defined and consistently used (Agar ranking 4, KWS 2) samples tested all came from lines not carrying the favorable convicilin gene signature mutations as identified, introduced and tested for certain lines. Further, the Agar ranking matched with the molecular results for the storage protein test so that this test for screening lines quickly with a visual read-out before further molecular screening is applied could be provided.

KWS 1 and KWS 3, both showing a vicilin to convicilin ratio of ~ 5:1 and both carrying at least one mutation (different pattern) in at least one convicilin gene, the mutation(s) representing signature mutations as identified (cf. **Tables 1** and **2** herein above) also showed a high Agar ranking score.

Our results suggest that genotype has influence on protein composition, as well as on technological-functional properties of pea proteins. This knowledge could be very useful in efforts to improve the quality of peas and pea protein products.

Strikingly, KWS line 1 and KWS line 3, both carrying signature mutations as identified to be correlated to a higher gelation pattern both indeed showed a vicilin to convicilin ratio of about 5:1 (see **Fig. 4** and **5** for KWS 1), whereas the reference line Proklam and a test lines KWS 2 and further lines tested in comparison, none of them having a signature mutation in a convicilin gene identified to be highly suitable herein, showed a significantly lower Agar ranking score and - at the same time on molecular level - a much lower vicilin to convicilin (each total protein +/- signal peptide) ration of 2:1 to ~ 3.5:1 and 4:1 at most.

### Example 6: DSC Analysis

For testing certain parameters, differential scanning calorimetry (DSC) can be applied, particularly to check whether a concentrated pea protein solution is suitable for use as a protein ingredient, and whether its proteins have a high or at least sufficient functionality. This test is thus useful in case the pea proteins with the favorably altered gelation pattern will be used together with other ingredients in compositions for alternative food, feed or for cosmetic purposes. Preferably, the denaturation enthalpy (which is the peak area of the DSC measurement) is usually at least 10 J/g protein, or more. To carry out the differential calorimetry measurement, at least 50 to 100 mg of a given pea protein concentrate sample with a known protein content are weighed (use a steel vessel) in a volume of 100 µl and closed pressure-tight. Another steel vessel is then filled with water. This second vessel serves as a reference value for the measurement. One suitable device is that can be used for the measurement is the Mettler Toledo Type DSC 1 Star. of the devices performes a temperature scan with a heating rate of 2 K/min. The scan starts at 25° C and,preferably, ends at 130° C for a usual measurement. Denaturation of the proteins in a specific temperature range are easily detacable in the DSC curve obtained as an endothermic peak, which is characterized by a peak start temperature, a peak maximum temperature, i.e., a temperature at the peak maximum, usually between 90° C and 125° C, and a peak end temperature. The peak temperature range, which is passed through between the peak start temperature and the peak end temperature, is preferably between 25° C and 40° C, in certain settings between 30° C and 35° C. The peak area of the endothermic peak in the DSC curve is thus a measure of the extent of denaturation, as it is known to one skilled in the art. Prticularly suitable concentrated plant protein solutionswill thus preferably be above 10 J/g protein, more preferably between 12 J/g and 30 J/g protein, whereinthe highest possible value is sought to be achieved.

### Example 7: Oscillation Rheology

To determine whether a concentrated pea protein isolate of this invention when in solution has sufficient aggregation behavior to function as a suitable protein ingredient for food applications, the pea protein concentrate solution is filled into a suitable steel vessel (e.g., beaker: C25 DIN system), usually between 10 and 15 ml. The vessel is then closed pressure-tight. The rheological properties can then be measured by means of the cylinder (C25 DIN system) which is positioned in the vessel containing the protein concentrate solution. Preferably, an absolutely pressure tight cylinder is used. Regarding relevant parameters, G' and G" are measured, i.e., the elastic and the viscous portion of the viscoelastic concentrate, wherein it is well understood that the two portions G' and G" change during the subsequent temperature cycles. The starting temperature is set to be 25° C. Next, a rapid heating with a heating rate between 3 K/min and 5 K/min takes place up to the relevant peak end temperature from the previous DSC measurement. Favorably, a short holding time step between 2 and 5 min at this temperature ensures that the pea protein concentrate or isolate was also completely exposed to this temperature. Subsequently, cooling is performed in a rapid way at a rate between about 3 K/min and 5 K/min. G' and G" are continuously recorded. In the region of the peak initial temperature for suitable pea protein concentrate solutions with an aggregation behavior suitable or sufficient for carrying out the method according to the invention, a strong increase in the G' values is usually observed during the heating phase. The pea protein concentrate or isolate solution is usually considered suitable for carrying out the method according to the invention if the storage modulus still increases during the heating phase by at least 6x, preferably 6x-12x, more preferably 7x to 13x, compared with the storage value at the beginning of the measurement, in particular at 25° C. The storage modulus G' will continue to increase during cooling until the gel is solidified. Again, the characteristics of aggregation behavior suitable for the methods according to the invention is the significant rise in G' during the heating phase in the range between the peak start temperature and the peak end temperature.

## Claims

1. A method of altering a gelation pattern of the pea protein ingredient of a *Pisum sativum* plant, the method comprising the following steps:
(i) providing at least one pea comprising at least one nucleic acid molecule comprising at least one modification of a nucleotide sequence of at least one gene, including the regulatory region thereof, wherein the at least one gene modified encodes a convicilin protein, wherein said at least one modification affects the gelation pattern of the pea protein ingredient, wherein said at least one modification affects at least one nucleotide sequence of a Psat06G0558100 reference gene (SEQ ID NO: 2) in comparison to the variant Psat06G0558100 reference gene (SEQ ID NO: 1) and/or of a Psat06G0558200 reference gene (SEQ ID NO: 66) in comparison to the variant Psat06G0558200 reference gene (SEQ ID NO: 65), or of a combination thereof;
(ii) optionally: cultivating the *Pisum sativum* plant to obtain at least one pea comprising a protein composition having an altered gelation pattern in comparison to a *Pisum sativum* plant not comprising the at least one modified convicilin gene, or the regulatory region thereof; and
(iii) obtaining a pea protein ingredient of said *Pisum sativum* plant, wherein saidpea protein ingredient obtained, preferably as intermediate, has an altered gelation pattern, wherein the altered gelation pattern is defined as an altered vicilin to convicilin ratio in a pea protein ingredient, preferably in a pea flour, in comparison to a reference vicilin to convicilin ratio of 2:1 to 4:1 (wt./wt.) of a pea protein ingredient, preferably a flour, of a reference *Pisum sativum* plant, wherein the altered vicilin to convicilin ratio is **characterized by** a decreased amount of convicilin in comparison to the amount measured for the reference *Pisum sativum* plant and thus an increased vicilin to convicilin ratio.

2. The method of claim 1, wherein the amount of convicilin in the pea protein ingredient, preferably in the pea flour, is reduced by at least 5%, at least 10%, at least 15%, at least 20%, at least 25%, preferably at least 30%, at least 35%, at least 40%, at least 45%, at least 50%, at least 55%, at least 60%, at least 70%, more preferably at least 75%, at least 80%, at least 85%, at least 90%, at least 95%, at least 100%, even more preferably at least 200%, at least 250%, at least 300%, at least 400%, at least 500%, and most preferably almost completely lost in comparison to the amount measured for the reference *Pisum sativum* plant.

3. The method of claim 1 or 2, further comprising a step of processing, preferably including a step of extraction, and/or purifying the pea protein ingredient of said *Pisum sativum* plant having an altered gelation pattern, wherein the processing preferably at least includes a step of dehulling, to obtain a protein flour, a protein concentrate, a protein isolate, a pea protein texturate, a pea protein powder, or a pea protein flake of a *Pisum sativum* plant.

4. The method of any of the preceding claims, wherein the convicilin protein has a sequence selected from any one of SEQ ID NOs: 121 and 122, or a sequence having at least 85%, 86%, 87%, 88%, 89%, 90%, 91%, 92%, 93%, 94%, 95%, 96%, 97%, 98%, or at least 99% sequence identity thereto, and/or wherein the vicilin protein has a sequence of SEQ ID NO: 123 or a sequence having at least 85%, 86%, 87%, 88%, 89%, 90%, 91%, 92%, 93%, 94%, 95%, 96%, 97%, 98%, or at least 99% sequence identity thereto.

5. A method of any of the preceding claims, wherein the at least one modification is a deletion, substitution, or an insertion, preferably a targeted substitution, of at least one nucleotide sequence of the at least one gene, including the regulatory region thereof, encoding the convicilin protein.

6. The method of claim 5, wherein the at least one modification is provided by a step of mutagenesis, wherein mutagenesis includes chemical mutagenesis, radiation mutagenesis, and genome editing, wherein genome editing includes editing by site-directed nucleases, including zinc-finger nuclease (ZFNs) systems, transcription activator-like effector nuclease (TALENs) systems, meganuclease systems and CRISPR/Cas systems, preferably wherein mutagenesis is resulting in a knock-down, more preferably a knock-out of at least one gene encoding convicilin.

7. A nucleic acid molecule comprising at least one modification of a nucleotide sequence of at least one gene, including the regulatory region thereof, the gene encoding a convicilin protein, wherein the modified nucleotide sequence is selected from any one of SEQ ID NOs: 3 to 64 or 1, having at least one modification in comparison to the reference gene sequence of SEQ ID NO: 2, and/or wherein the modified nucleotide sequence is selected from any one of SEQ ID NOs: 67 to 120, or 65 having at least one modification in comparison to the reference gene sequence of SEQ ID NO: 66, or any combination the aforementioned modifications of SEQ ID NOs: 3 to 64 and/or SEQ ID NOs: 67 to 120, or a nucleotide sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or at least 99% sequence identity to the respective sequence provided that the respective signature mutation as defined in Table 1 and Table 2 is still present, or a nucleic acid molecule comprising at least one modification of a nucleotide sequence of at least one gene, including the regulatory region thereof, the gene encoding a convicilin protein, wherein the modified nucleotide sequence comprises at least one signature mutation as defined in Table 1 and Table 2 in comparison to the reference gene sequence of SEQ ID NO: 2 or SEQ ID NO: 66, respectively.

8. An expression construct or a vector comprising at least one modified nucleotide sequence of at least one convicilin gene and/or of the regulatory regions thereof of claim 5.

9. A protein flour, a protein concentrate, a protein isolate, a pea protein texturate, a pea protein powder, or a pea protein flake of a *Pisum sativum* plant, having an altered gelation pattern, wherein the *Pisum sativum* plant comprises at least one modification of a nucleotide molecule in a nucleotide sequence of at least one convicilin gene, including the regulatory sequence thereof, wherein the nucleotide molecule is as defined in any one of claims 1 to 3 and 5 to 7, and/or wherein the *Pisum sativum* plant, or a cell thereof, comprises at least one expression construct or at least one vector of claim 8, or wherein the *Pisum sativum* plant comprises at least one modified convicilin protein of claim 4.

10. A pea protein mixture or a pea protein composition comprising a protein flour, a protein concentrate, a protein isolate, a pea protein texturate, a pea protein powder, or a pea protein flake of a *Pisum sativum* plant of claim 9 and additionally comprising at least one further additive and/or ingredient.

11. An alternative consumable or a cosmetic composition or product, or a hybrid composition or product, being selected from a solid and a semi-solid alternative consumable or cosmetic composition or product, comprising at least one protein flour, a protein concentrate, a protein isolate, a pea protein texturate, a pea protein powder, or a pea protein flake of a *Pisum sativum* plant claim of 9, or comprising at least one mixture of claim 10, wherein the alternative consumable or an alternative cosmetic composition has an improved gelation in comparison to a reference material.

12. The alternative consumable or the cosmetic composition or product, or the hybrid composition or product thereof of claim 11, wherein the composition or product has a pH in the range of about 3.5 to about 8.5, preferably a pH from about 4.5 to about 8.

13. A method of producing a protein flour, a protein concentrate, a protein isolate, a pea protein texturate, a pea protein powder, or a pea protein flake of a *Pisum sativum* plant of claim 9, or of a mixture or composition of claim 10, comprising the steps of:
(i) providing at least one pea from a *Pisum sativum* plant having an altered gelation pattern as defined in any one of claims 1 to 6, or comprising at least one nucleotide molecule of claim 7, or comprising at least one expression construct or vector of claim 8; and
(ii) optionally: dehulling the at least one pea;
(iii) milling the at least one pea to obtain a flour;
(iv) extracting at least one protein fraction, optionally: also including at least one starch fraction and/or at least one fat fraction, preferably wherein the extracting step is a dry extraction method, or wherein the extracting step is a wet extraction method; and
(v) optionally: further processing and/or purifying the extracted protein fraction and thus obtaining an enriched and/or purified fraction selected from the group consisting of: protein flour, a protein concentrate, a protein isolate, a pea protein texturate, a pea protein powder, or a pea protein flake of a *Pisum sativum* plant.

14. A method of screening and/or selecting a *Pisum sativum* plant, wherein a pea protein ingredient obtained from said *Pisum* plant, preferably as intermediate, has an altered gelation pattern as defined in any one of claims 1 to 6, or wherein said plant comprises at least one nucleotide molecule of claim 7, wherein the method comprises:
(i) using at least one probe or at least one primer capable of identifying a signature mutation of claim 7;
(ii) identifying and optionally: obtaining at least one *Pisum sativum* plant having at least one modification of a nucleotide sequence of at least one gene, including the regulatory region thereof, the gene encoding a convicilin protein, as defined in any one of claims 1 to 7, or a combination of more than one modification.

15. A use of a protein flour, a protein concentrate, a protein isolate, a pea protein texturate, a pea protein powder, or a pea protein flake of a *Pisum sativum* plant as defined in claim 9, or a use of a mixture as defined in claim 10 for preparing an alternative consumable composition or an alternative cosmetic composition, preferably wherein the consumable composition is a solid or semi-solid alternative consumable composition or an alternative cosmetic composition.
